# EUROPÄISCHE PATENTSCHRIFT

(11) **EP 3 914 196 B1**
(45) Veröffentlichungstag und Bekanntmachung des Hinweises auf die Patenterteilung: **01.01.2025**
(21) Anmeldenummer: 20702265.8
(22) Anmeldetag: 24.01.2020
(51) Int. Cl.: A61F 2/66, A61F 2/68, A61F 2/50

(54) **PROTHESENFUSSEINSATZ**
PROSTHETIC FOOT COMPONENT
INSERT DE PROTHÈSE DE PIED

(30) Priorität: 25.01.2019 DE 102019101835
(43) Veröffentlichungstag der Anmeldung: 01.12.2021
(73) Patentinhaber: Ottobock SE & Co. KGaA, 37115 Duderstadt (DE)
(72) Erfinder: PUSCH, Martin, 37115 Duderstadt (DE); MOENICKE, Carsten, 37115 Duderstadt (DE); KRENZ, Hannes, 01309 Dresden (DE)
(74) Vertreter: Gramm, Lins & Partner Patent- und Rechtsanwälte PartGmbB
(86) Internationale Anmeldenummer: PCT/EP2020/051783
(87) Internationale Veröffentlichungsnummer: WO 2020/152341

(56) Entgegenhaltungen:
- DE-A1- 102014 010 938
- DE-A1- 102015 101 746
- DE-A1- 102015 101 746
- US-A1- 2015 190 247
- US-A1- 2015 190 247
- US-A1- 2015 328 020
- US-A1- 2015 328 020

## Beschreibung

Die Erfindung betrifft einen Prothesenfußeinsatz mit einer proximalen Befestigungseinrichtung zum Festlegen des Prothesenfußeinsatzes an einer Proximalkomponente, einem distal zu der Befestigungseinrichtung angeordneten und mit der Befestigungseinrichtung gekoppelten Halter, einem elastischen Fersenelement, das an dem Halter angeordnet ist und einer Hauptfeder, die sich in einen Vorderfußbereich erstreckt und mit dem Halter gekoppelt ist.

Prothesenfußeinsätze sind Teil einer prothetischen Versorgung, beispielsweise bei Unterschenkelamputierten. Prothesenfußeinsätze können zur Erzielung einer möglichst natürlichen Optik sowie zur Bereitstellung einer weiteren Funktionalität mit einem Überzug oder einer Prothesenkosmetik versehen sein, die aus einem Kunststoff ausgebildet sein kann. Die Prothesenfußeinsätze können an einem Knöchelgelenk oder gelenklos an einem Unterschenkelrohr oder einem Unterschenkelschaft befestigt sein. Die Befestigungseinrichtung ist in der Regel als sogenannter Pyramidenadapter ausgebildet, über den eine Vielzahl von Einstellungen und Ausrichtungen des Prothesenfußeinsatzes relativ zu der Proximalkomponente, also dem Unterschenkelrohr, dem Prothesenschaft oder dem Knöchelgelenk eingestellt und fixiert werden können. Die Befestigungseinrichtung ist an einem Halter befestigt, an dem wiederum eine sich in Vorfußrichtung erstreckende Feder, beispielsweise eine Vorfußfeder oder Dachfeder angeordnet sein kann. Um einen Stoß bei einem Fersenauftritt abzudämpfen, ist ein elastisches Fersenelement vorgesehen, das an dem Halter befestigt ist, gegebenenfalls unter Einschaltung von Zwischenstücken. Beispiele für einen Protheseneinsatz sind in der EP 2 420 212 A1, der EP 1 976 463 A1, der US 2005/0038525 A1 oder der EP 2 688 522 B1 beschrieben.

Die US 2015/190247 A1 betrifft einen Prothesenfußeinsatz mit einer Sohlenstruktur, die in einem Zehenbereich von unten gesehen eine konvexe Wirkung aufweist, die im Mittelfußbereich in eine konkave Wölbung übergeht und sich bis zu einem Fersenbereich erstreckt. An einer Stützstruktur sind proximale Anschlussmittel angeordnet. Zwischen der Stützstruktur und der Sohlenstruktur ist ein elastisches Verbindungselement angeordnet, das einen horizontalen Schlitz aufweist, in den die Sohlenstruktur eingeführt ist. Zwischen der Sohlenstruktur und der Stützstruktur kann eine Steuerfeder angeordnet sein, die mit ihrem fersengerichteten Arm in das elastische Verbindungselement hineinragt. US 2015/190247 beschreibt einen Prothesenfußeinsatz gemäß dem Oberbegriff des Anspruchs 1.

Die DE 10 2015 101 746 A1 beschreibt einen Prothesenfuß mit einem Gehäuse, an dem ein Anschluss für eine Unterschenkelprothese anordenbar ist. Das Gehäuse ist mit dem Vorfuß durch zumindest ein Vorfuß-Federelement verbunden. Der Vorfuß und die Ferse sind durch zumindest ein Sohlen-Federelement verbunden. Die Ferse und das Gehäuse sind durch zumindest ein Fersen-Federelement verbunden. Das Sohlen-Federelement erstreckt sich über den Vorfuß hinaus und bildet einen Zehenbereich aus, an dessen vorderem Ende ein Band angreift, das sich entlang der Unterseite des Sohlen-Federelementes bis zu der Ferse und durch diese hindurch bis zu dem Fersenbereich des Gehäuses erstreckt. Das Band ist zumindest einseitig mit einer Vorspanneinrichtung verbunden, durch welche zwischen einer Einspannstelle an dem Gehäuse und an dem Zehenbereich eine Vorspannung auf ein Dämpfungselement aufbringbar ist.

Die US 2015/0328020 A1 beschreibt unter anderem eine Ausführungsformen eines Prothesenfußes, in der ein Befestigungsteil zum Festlegen an einem Unterschenkelschaft oder einem Prothesenkniegelenk an einem ersten, C-förmigen, flexiblen Bauteil befestigt ist. Ein flexibles Element ist zwischen dem flexiblen Bauteil und einer Sohlenfeder angeordnet und im Fersenbereich über zwei einander gegenüberliegende Schraubenfedern gegenüber dem flexiblen Bauteil und der Sohlenfeder abgestützt.

Die DE 10 2014 010 938 A1 betrifft einen Prothesenfuß mit einem Fußteil, einem proximalen Anschlussmittel, das schwenkbar mit dem Fußteil verbunden ist und einer Verstelleinrichtung, mit der das Fußteil relativ zu dem Anschlussmittel verstellbar ist.

Die proximalen Anschlussmittel sind als ein verschwenkbarer Pyramidenadapter dargestellt.

Problematisch bei Prothesenfußeinsätzen aus dem Stand der Technik ist der gegebenenfalls benötigte Bauraum, ein unbefriedigendes Einsinkverhalten, ein ungleichmäßiges Abrollverhalten und Schwierigkeiten beim Ausgleichen von Unebenheiten. Darüber hinaus sind teilweise komplexe Formgebungen notwendig, die die Herstellkosten erhöhen und Schwierigkeiten bei der optimalen Materialausnutzung bereiten.

Aufgabe der vorliegenden Erfindung ist es daher, einen Prothesenfußeinsatz bereitzustellen, der bei einem kleinen Bauraum eine optimale Materialausnutzung bei einem einfachen Aufbau ermöglicht.

Erfindungsgemäß wird diese Aufgabe durch einen Prothesenfußeinsatz mit den Merkmalen des Hauptanspruchs gelöst. Vorteilhafte Ausgestaltungen und Weiterbildungen der Erfindung sind in den Unteransprüchen, der Beschreibung sowie den Figuren offenbart.

Der Prothesenfußeinsatz mit einer proximalen Befestigungseinrichtung zum Festlegen des Prothesenfußeinsatzes an einer Proximalkomponente, einem distal zu der Befestigungseinrichtung angeordneten und mit der Befestigungseinrichtung gekoppelten Halter, einem elastischen Fersenelement, das an dem Halter angeordnet, insbesondere befestigt ist und eine Hauptfeder, die sich in einen Vorderfußbereich erstreckt und mit dem Halter gekoppelt ist, sieht vor, dass die Hauptfeder an dem Fersenelement zwischen einer proximalen Fersenkomponente und einer distalen Fersenkomponente gelagert ist. Der Halter und die Befestigungseinrichtung können einstückig gemeinsam ausgebildet oder als separate Komponenten aneinander festgelegt sein, beispielsweise verschraubt, verklebt, verrastet oder verschweißt. Das Fersenelement ist dem Halter zugeordnet, insbesondere angeschraubt, angeklebt, aufgesteckt, verrastet, verschweißt oder auf eine andere Art und Weise Kräfte und/oder Momente übertragend an dem Halter angeordnet oder festgelegt sein. Das Fersenelement ist somit zweigeteilt, wobei eine distale Fersenkomponente bodenseitig oder distal zu der Hauptfeder wirksam ist, während eine obere, proximale Fersenkomponente in Richtung auf den Halter wirksam ist. Dadurch ist es möglich, eine Reihenschaltung der beiden Fersenkomponenten bei einer Fersenbelastung zu realisieren, wodurch beide Fersenkomponenten wirksam werden. Dadurch ist es möglich, die Stoßbelastung bei einem Fersenauftritt angenehm zu dämpfen. Während der gesamten Druckbelastung des Fersenelementes während des Gehens oder Stehens wirken beide Fersenkomponenten zusammen. Bei einer Vorfußbelastung wirkt die Hauptfeder zusammen mit der distalen Fersenkomponente, wodurch sich eine Doppelnutzung und dadurch eine verbesserte Materialausnutzung ergeben. Der Prothesenfußeinsatz kann als Basis für sonstige Aufbauten wie ein mechatronisches Gelenk, ein ML-Adapter, ein Adapter zum Einstellen der Absatzhöhen, eine hydraulische Gelenkeinheit oder dergleichen eingesetzt werden. Die Erfindung sieht vor, dass das Fersenelement über zumindest ein Spannelement mit dem Halter gekoppelt ist. Das Spannelement kann zugstarr und flexibel ausgebildet sein, ebenso kann es als elastisches und flexibles Element, z.B. als ein Gurt oder ein Band oder als nachgiebiges Element oder Elastomerelement ausgebildet sein. Das fersenseitige Spannelement kann die Fersenkomponente gegen ein seitliches Ausweichen stabilisieren, insbesondere, wenn das Spannelement medial und lateral an der Außenseite der Fersenkomponenten entlang geführt ist. Über das Spannelement kann eine Vorkompression der Fersenkomponenten oder des gesamten Fersenelementes bewirkt werden, wodurch eine zum Balancieren in anterior-posterior Richtung erforderliche Vorspannung erreicht wird. Das Spannelement kann verstellbar, insbesondere verkürzbar oder verlängerbar an dem Halter und/oder dem Fersenelement oder einem daran angeordneten oder dem zugeordneten Aufnahmeteil gelagert sein. Alternativ kann durch Einleger oder Distanzstücke die Vorspannung variiert werden. Das Spannelement kann als geschlossene Schlaufe, als Stab, als Seil- oder Bandverbindung oder Spange ausgebildet sein. Eine Veränderung der Vorspannung kann durch Auswechseln der Schlaufe, des Bandes, des Seils des Stabes oder der Spange und Einlegen eines anderen Spannelementes mit einem abweichenden Umfang, einer abweichenden Länge oder eine abweichenden Spannweite erreicht werden. An dem Halter kann eine Aufnahme oder eine Nut ausgebildet sein in die das Spannelement eingelegt wird. Gegen eine unbeabsichtigte Entfernung des Spannelementes von dem Halter kann eine mechanische Sicherung über der Nut angeordnet sein, die beispielsweise Teil des Befestigungselementes sein kann. Veränderungen in der Vorspannung können auch durch Polster oder Einlegeelemente erreicht werden, die zwischen dem Spannelement und dem Fersenelement und/oder dem Halter angeordnet sind. Insbesondere bei einer Ausgestaltung des Spannelementes als ein Gurt kann durch eine oder mehrere Einleger oder Distanzelemente eine Verschleißminderung erreicht werden, da ein direkter Kontakt mit dem starren Halter vermieden wird. Das Spannelement hält das Fersenelement in einer unbelasteten Ausgangsposition in einer komprimierten Stellung. Wird eine hohe Fersenlast aufgebracht, kann das Spannelement ohne Zugspannung an dem Halter und/oder dem Fersenelement befestigt sein. Wird eine Vorfußlast aufgebracht, bildet das Spannelement das Widerlager für die Hauptfeder und die distale Fersenkomponente. Bei einer Vorderfußbelastung oder einer Zehenlast wird die distale Fersenkomponente gegen die Hauptfeder gespannt und mit einer Kraft beaufschlagt, so dass sie gegen die Feder gedrückt wird. Bei einem zugstarren Material handelt es sich um ein Material, das keine oder nur eine geringfügige Dehnung zulässt, also eine hohe Dehnsteifigkeit aufweist. Der Elastizitätsmodul ist größer als fünf Kilonewton pro Quadratmillimeter. Das Spannelement kann auch eine gewisse Elastizität aufweisen und als Band oder anders geformtes Elastomerelement ausgebildet sein.

Die distale Fersenkomponente kann in einer Variante der Erfindung starr ausgebildet sein, wobei die proximale Fersenkomponente an dem Halter und der Hauptfeder festgelegt ist. Die proximale Fersenkomponente ist Druckkräfte übertragend und Zugkräfte übertragend an dem Halter und der Hauptfeder festgelegt, beispielsweise festgeklebt, verschweißt, angespritzt, angegossen, additiv gefertigt oder formschlüssig gekoppelt, beispielsweise durch Schrauben, Bolzen, Clips, Bajonettverschlüsse Schwalbenschwanzführungen, Winkelschienen oder andere Befestigungselemente, wodurch insbesondere eine reversible Festlegung an dem Halter und der Hauptfeder und eine Austauschbarkeit erzielt werden kann. Die distale Fersenkomponente wirkt dabei sowohl bei einer Vorderfußbelastung als auch bei einer Fersenbelastung.

Vorteilhafterweise sind die Fersenkomponenten gleichartig aufgebaut, sodass sie ausgetauscht werden können. Dadurch ist es möglich, unterschiedliche Federcharakteristiken bei Vorfußbelastung mit denselben Fersenkomponenten zu ermöglichen, indem die beiden Komponenten vertauscht werden. Die distale Fersenkomponente kann härter als die proximale Fersenkomponente ausgebildet sein, um unterschiedliche Einfederverhalten bei Vorderfußbelastung und Fersenbelastung einstellen zu können. Bei einer Fersenbelastung wirken beide in Reihe angeordneten Fersenkomponenten, bei einer Vorderfußbelastung wirkt nur die oder ganz überwiegend die distale Fersenkomponente.

Die Hauptfeder ist in einer Ausgestaltung der Erfindung zwischen der proximalen und der distalen Fersenkomponente eingebettet, vorteilhafterweise trennt die Hauptfeder die proximale Fersenkomponente von der distalen Fersenkomponente. Die Hauptfeder liegt zwischen den beiden Fersenkomponenten und kann mit diesen integral ausgebildet oder unlösbar verbunden sein. Ebenfalls ist eine Festlegung an der Hauptfeder oder eine Anformung, einstückige Ausgestaltung in einem additiven Herstellverfahren oder eine andere dauerhafte Verbindung zwischen den Fersenkomponenten und der Hauptfeder in einer Weiterbildung verwirklicht. Sind die Fersenkomponenten nur in Druckrichtung miteinander gekoppelt, besteht die Möglichkeit, dass bei einer Vorfußbelastung ausschließlich die distale Fersenkomponente mit der Hauptfeder zusammenwirkt. Sind beide Fersenkomponenten sowohl zugkraftübertragend als auch druckkraftübertragend mit der Hauptfeder gekoppelt, beispielsweise durch eine formschlüssige Verriegelung, durch Verkleben oder ähnliches kann durch unterschiedliche Dehnungseigenschaften bei Zug- und Druckbelastung eine unterschiedliche Abstimmung hinsichtlich der Federdämpfereigenschaften bei einer Vorfußbelastung und bei einer Fersenbelastung erreicht werden. Das proximale Fersenelement ist bevorzugt fest an dem Halter fixiert, vorteilhafterweise lösbar fixiert, um eine Anpassung an unterschiedliche Nutzer oder unterschiedliche Einsatzbedingungen vornehmen zu können. Ebenso ist das distale Fersenelement vorzugsweise auswechselbar an der Hauptfeder angeordnet oder gegenüber der Hauptfeder vorgespannt, sodass die distale Fersenkomponente stets an der bodenseitigen Oberfläche der Hauptfeder gehalten ist.

Bevorzugt ist die distale Fersenkomponente härter als die proximale Fersenkomponente, sodass ein weicher Auftritt bei einer Fersenlast mit einer vergleichsweise harten Feder aus der Kombination der Hauptfeder mit der distalen Fersenkomponente erreicht wird. Dadurch lässt sich im Bereich der terminalen Standphase eine hohe Energierückführung einer gleichzeitig hohen Kontrollierbarkeit der Abrollbewegung erreichen. Die Hauptfeder wirkt bei einer Vorfußlast mit der distalen Fersenkomponente zusammen, bei einer Fersenlast wirken vordringlich die beiden Fersenkomponenten des elastischen Fersenelementes, sodass eine weiche Ferse beim Stehen und beim Aufsetzen der Ferse erreicht wird. Dadurch wird insbesondere bei einem gelenkigen Prothesenfuß eine schnelle Plantarflexion erreicht, wodurch eine erhöhte Kniestabilität nach dem vollständigen Aufsetzen des Fußes erreicht wird.

Die Hauptfeder ist in einer Ausgestaltung der Erfindung nicht Zugkräfte übertragend mit der proximalen Fersenkomponente gekoppelt, um die Fersenkomponenten nur auf Druck belasten zu müssen. Dadurch wird die Haltbarkeit der Fersenkomponenten, die insbesondere als Schaumelemente oder aus einem Elastomer ausgebildet sein können, vergrößert.

Die Hauptfeder ist an einer Ausgestaltung der Erfindung als Blattfeder ausgebildet, insbesondere als eine gerade Blattfeder, die zwischen den beiden Fersenkomponenten angeordnet ist, vorteilhafterweise beide Fersenkomponenten voneinander trennt. Die Ausgestaltung auf einer geraden Blattfeder hat den Vorteil, dass die Herstellung außerordentlich einfach ist, insbesondere wenn die Hauptfeder aus einem faserverstärkten Kunststoff hergestellt ist. Die Federn können aus Glasfasern, Karbonfasern, Aramid, Kevlar, Dyneema etc oder aus Kombinationen davon in einer Matrix eingebettet hergestellt werden. Die Hauptfeder kann vergleichsweise steif ausgelegt werden, sodass sich die Haltbarkeit erhöht, da eine steife Federauslegung der Hauptfeder über die distale Fersenkomponente bei Vorfußbelastung kompensiert wird, sodass eine ausreichende Nachgiebigkeit auch bei einer Vorfußbelastung erreicht wird.

Eine Weiterbildung der Erfindung sieht vor, dass der Halter einen in Richtung auf den Vorderfußbereich über das Befestigungselement hervorstehenden Vorsprung aufweist, der sich auf der Hauptfeder abstützt. Die Abstützung muss nicht unmittelbar auf der Hauptfeder erfolgen, es können auch Zwischenstücke, ein oder mehrere Gelenke, ein oder mehrere Elastomerelemente oder ähnliches zwischen dem Halter und der Hauptfeder vorgesehen sein. Über den Vorsprung ist es möglich, den Ort der Krafteinleitung zu variieren. Während des Gehens, beim Rollover, oder beim Stehen, wenn der Kraftvektor vor die Senkrechte durch das Befestigungselement wandert, werden Kräfte von dem Halter auf die Hauptfeder eingeleitet. Die Hauptfeder stützt sich im Vorderfußbereich auch auf dem Boden ab, sodass durch die Krafteinleitung über den Vorsprung die Biegebelastung der Hauptfeder im Mittelfußbereich eingeleitet werden kann.

In einer Weiterbildung stützt sich der Halter an zwei in Längserstreckung der Hauptfeder zueinander beabstandeten Punkten auf der Hauptfeder ab, entweder unmittelbar oder aber über eine Zwischenfeder oder Zwischenplatte oder eine Spange, die sich bevorzugt an zwei zueinander beabstandeten Punkten auf der Hauptfeder abstützt. Die Abstützung der Zwischenfeder oder Zwischenplatte auf der Hauptfeder kann beispielsweise über zwei Klebepunkte oder ähnliches erreicht werden. Wird eine Krafteinleitung über zwei Punkte zwischen den Fersenkomponenten und dem vorderen Auflagepunkt oder dem vorderen Abstützpunkt am Boden eingeleitet, beispielsweise über die Verlagerung einer Zwischenfeder oder Zwischenplatte auf zwei Punkten an der Hauptfeder, findet eine Vier-Punkt-Biegung statt, wenn eine Vorfußbelastung vorliegt, wodurch das maximale Biegemoment in der Hauptfeder wesentlich reduziert wird. Die Zwischenfeder ist in einer Ausführung als eine gerade Blattfeder ausgebildet, die auf zwei Lagerelementen auf der Hauptfeder gelagert ist, sodass bei einer beispielsweise zentralen Krafteinleitung über den Halter auf die Zwischenfeder die Kraft an zwei Stellen in die Hauptfeder eingeleitet wird. Im Unterschied zu der Zwischenfeder ist die Zwischenplatte im Wesentlichen biegestarr oder biegesteif ausgebildet, wird nachfolgend von Zwischenfeder gesprochen, ist darunter auch eine starre oder im Wesentlichen starre Platte zu verstehen, wenn nicht ausdrücklich auf die elastischen Eigenschaften Bezug genommen wird.

An dem Fersenelement kann ein Führungselement befestigt sein, das an der Hauptfeder gelagert ist. Das Führungselement kann gelenkig an der Hauptfeder gelagert sein, beispielsweise im frontalen Endbereich der Hauptfeder. Alternativ ist das Führungselement als eine gerade Blattfeder ausgeführt, beispielsweise aus einem Faserverbundwerkstoff. Das Führungselement in Gestalt einer Blattfeder kann beispielsweise sohlenseitig und im Bereich der Zehe mit der Hauptfeder verbunden, beispielsweise verschraubt sein und ist an dem rückwärtigen Ende mit dem Fersenelement gekoppelt. Die distale Fersenkomponente kann unmittelbar an dem Führungselement festgelegt sein, alternativ kann an dem Führungselement eine Aufnahme angeordnet sein, über die eine Kopplung der distalen Fersenkomponente mit dem Führungselement erfolgt. Auch bei einer Ausgestaltung des Führungselementes als Blattfeder soll das Führungselement keine oder nur eine geringe Federwirkung ausüben. Dazu ist es vorgesehen, dass das Führungselement sehr dünn ausgebildet ist, wobei in dem ursprünglich montierten Zustand durch die Federwirkung des Führungselementes keine oder nur eine geringe Kompressionswirkung auf das Fersenelement ausgeübt wird. Die Befestigung und Ausgestaltung des Führungselementes sperrt oder schränkt eine medial-lateral-Bewegung des Fersenelementes während der Benutzung des Prothesenfußeinsatzes ein, sodass die Orientierung und Positionierung des Fersenelementes relativ zu dem Halter und zu der Hauptfeder stabilisiert wird. Darüber hinaus stellt das Führungselement die Ausrichtung des Fersenelementes zu der Hauptfeder und zu dem Befestigungselement sicher, was auch bei einer Verwendung einer Fußkosmetikhülle vorteilhaft ist. Das Führungselement lässt eine Kompression und Expansion des Fersenelementes zu, es werden nur geringe oder gar keine Rückstellkräfte durch das Führungselement in proximal-distal-Richtung ausübt.

Das Spannelement ist in einer Ausgestaltung der Erfindung distal der distalen Fersenkomponente geführt, um ein Widerlager gegen eine Kompression über die Hauptfeder bei einer Vorfußbelastung zu biegen. Das Spannelement kann distal, beispielsweise an dem Führungselement befestigt oder distal um das Führungselement herumgelegt sein. Bei einer offenen Ausgestaltung des Spannelementes oder bei einer Ausgestaltung mit zwei Spannelementen, eines medial und eines lateral dem Fersenelement geführt, können die Spannelemente oder das Spannelement einzeln an der Unterseite oder an dem distalen Ende der distalen Fersenkomponente befestigt sein.

An der distalen Fersenkomponente oder an dem Führungselement kann ein Sohlenelement angeordnet sein, das eine Fersenkontur ausbilden kann. Das Sohlenelement bildet einen distalen Abschluss des Fersenelementes und kann distal zu dem Führungselement und daran angeordnet sein. Das Sohlenelement kann das Führungselement aufnehmen, beispielsweise in einem Schlitz oder in einer Nut. Das Führungselement kann in dem Sohlenelement festgelegt sein, beispielsweise eingeschraubt, eingegossen, eingeklebt oder dergleichen oder nur eingesteckt. Das Sohlenelement kann eine distale Kontur aufweisen, die an den jeweiligen Nutzer angepasst ist oder anpassbar ist. Für transfemorale Amputationspatienten kann eine andere Kontur notwendig sein als für einen Patienten mit einer transtibialen Amputation. Das Spannelement kann zur Verschleißminimierung in dem Sohlenelement eingespritzt oder integriert sein. Das Sohlenelement muss sich nicht über die gesamte Länge des Prothesenfußeinsatzes erstrecken, vorteilhafterweise befindet sich das Sohlenelement nur im Fersenbereich, um die mechanische Wirkungsweise der übrigen Komponenten wie Halter, Hauptfeder, Zwischenfeder oder Zwischenplatte und Fersenelement nicht zu beeinträchtigen. Das Sohlenelement kann aufgeklebt oder mit Formschlusselementen versehen und an dem Führungselement aufgeklipst oder auf eine andere Art und Weise befestigt sein. An dem Sohlenelement können Formschlusselemente ausgebildet sein, um die distale Fersenkomponente aufzunehmen und mechanisch zu sichern. Distal kann an dem Sohlenelement eine Aufnahme oder ein Befestigungselement angeordnet sein, um ein Konturelement an dem Sohlenelement anzubringen, um die Kontur des Sohlenelementes an die unterschiedlichen Nutzer anpassen zu können. An der Außenseite des Sohlenelementes können Vorsprünge oder Befestigungseinrichtungen angeordnet sein, die einen formschlüssigen Eingriff mit Vorsprüngen oder Hinterschnitten in einer Fußkosmetik ermöglichen.

In einer Ausgestaltung ist der Halter über ein Gelenk, das als Scharnier oder als Feder, beispielsweise eine Blattfeder ausgebildet sein kann, mit der Hauptfeder oder der Zwischenfeder oder einer Zwischenplatte gekoppelt. Das Gelenk ist vorteilhafterweise zentral, also im Mittelbereich der Längserstreckung der Hauptfeder angeordnet. An das Gelenk oder ein Federblech ist an dem Halter, der vorteilhafterweise formstabil ausgebildet ist, befestigt. Vorteilhafterweise ist das Gelenk verdrehfest sowohl an dem Halter als auch an der Zwischenfeder oder der Zwischenplatte oder der Hauptfeder festgelegt. Über das Gelenk beziehungsweise die Festlegung des Gelenkes an dem Halter und der Zwischenfeder oder Zwischenplatte oder der Feder wird eine Verschiebung des Halters relativ zu der Hauptfeder verhindert. Weiterhin wird durch ein Federblech oder ein Gelenk eine geringfügige Verdrehung oder Rotation in der Frontalebene ermöglicht, wie sie bei einer Vorderfußbelastung auftritt. Eine Ausgestaltung des Gelenks als ein Federblech wird die Krafteinleitung bei einer Vorderfußbelastung auf eine große Stützfläche verteilt, wodurch eine Flächenpressung vermieden wird. Dadurch werden die Zwischenfeder oder die Hauptfeder gegen eine hohe Druckbelastung geschützt, sodass die Haltbarkeit der jeweiligen Feder erhöht wird. Darüber hinaus stellt die Anbindung des Halters über ein Federblech oder ein Scharniergelenk eine dauerhafte und verschiebungsfreie Verbindung des Halters und damit des Befestigungselementes mit der Hauptfeder sicher. Eine Anordnung des Gelenkes oder der Krafteinleitung über ein Federblech im Mittelfußbereich hilft, die Federeigenschaften der Hauptfeder und der Zwischenfeder, soweit sie vorhanden ist, optimal auszunutzen.

An dem frontalen Ende der Hauptfeder kann eine Zehenfeder oder ein Zehenelement angeordnet sein, wodurch eine Anpassung an unterschiedliche Fußgrößen durch einfaches Anordnen, insbesondere Anschrauben einer Zehenfeder oder eines Zehenelementes an dem frontalen Ende der Hauptfeder ermöglicht wird. Darüber hinaus kann durch die auswechselbare Befestigung der Zehenfeder oder des Zehenelementes an der Hauptfeder eine Anpassung an einen rechten Prothesenfußeinsatz oder einen linken Prothesenfußeinsatz vorgenommen. Dadurch erhöht sich die Anzahl der Gleichteile, die für eine rechtseitige oder linksseitige Anbindung des Prothesenfußeinsatzes verwendet werden können. Somit werden gleichartige zentrale Blattfedern als Hauptfeder sowie entsprechende Fersenelemente sowohl für einen rechten als auch für einen linken Prothesenfußeinsatz verwendet, eine Anpassung an unterschiedliche Fußgrößen oder eine rechte oder linke Verwendung wird durch das Zehenelement oder eine Zehenfeder vorgenommen. Statt einer lösbaren Ausgestaltung kann auch eine unlösbare Verbindung erfolgen, beispielsweise durch Verkleben. Unlösbar heißt, dass mit den gleichen Komponenten keine erneute Verbindung möglich ist. Darüber hinaus ist es möglich, mit der Zehenfeder oder dem Zehenelement die sohlenseitige Kontur zu beeinflussen und das Energiemanagement nach maximaler Vorfußbelastung einzustellen. Über die Eigenschaften der Zehenfeder oder des Zehenelementes kann eine weitere Anpassung an den jeweiligen Prothesenfußeinsatznutzer erfolgen. Das Zehenelement kann klappbar oder verschwenkbar zu der Hauptfeder ausgebildet oder an dem Prothesenfußeinsatz angeordnet sein.

Die Fersenkomponenten bestehen vorteilhafterweise aus zumindest einem Schaumwerkstoff, Hohlkörper, Elastomerelement, Carbonelement, Elastomerelement mit Kavität als Pumpkammer und/oder Schraubenfederelementen. In dem Fersenelement oder zwischen sich zueinander verlagernden Komponenten des Prothesenfußeinsatzes kann zumindest eine Pumpeinrichtung oder ein Pumpelement angeordnet sein, beispielsweise um ein Unterdruck in einer proximalen Prothesenkomponente zu erzeugen. Die Pumpe kann in einer Fersenkomponente integriert sein oder als separates Bauteil oder separate Baugruppe zwischen dem Halter und der Hauptfeder, der Hauptfeder und dem Führungselement und/oder dem Halter und dem Führungselement angeordnet sein und durch die entsprechenden Relativverlagerungen angetrieben werden. Parallel zu der Pumpe kann eine Rückstellfeder angeordnet sein, die stärker als das zu erzeugende Vakuum ausgelegt ist, um eine Rückstellung in einen Ausgangszustand zu ermöglichen.

Die Federn, insbesondere die Hauptfeder und die Zwischenfeder sind vorteilhafterweise als gerade Blattfedern ausgebildet, wodurch sich eine preiswerte Fertigung erzielen lässt und die Materialeigenschaften optimal ausgenutzt werden.

Eine Weiterbildung der Erfindung sieht vor, dass zwischen dem Halter und der Hauptfeder ein Dämpfer oder ein Aktuator angeordnet ist. Über den Dämpfer ist es möglich, eine Relativbewegung des Halters zu der Hauptfeder zusätzlich zu beeinflussen und eine weitere Möglichkeit zur Anpassung und Einstellung an unterschiedliche Gangsituationen, Gehgeschwindigkeiten, Einsatzgebiete und/oder Patienten bereitzustellen. Alternativ zu einer Ausgestaltung als Dämpfer, insbesondere als Hydraulikdämpfer oder Pneumatikdämpfer, kann zwischen dem Halter und der Hauptfeder ein Aktuator angeordnet sein, über den die relative Position des Halters zu der Hauptfeder einstellbar ist. Dadurch können beispielsweise Anpassungen an unterschiedliche Absatzhöhungen für eine einmalige Einstellung oder fortlaufende Einstellungen während des Gehens an unterschiedliche Gehgeschwindigkeiten, Belastungen oder Gehsituationen vorgenommen werden. Der Aktuator ist insbesondere als Motor ausgebildet und kann auch im Generatorbetrieb als Dämpfer eingesetzt werden. Der Dämpfer kann zudem mit einer Verstelleinrichtung gekoppelt sein, um die Ventile innerhalb des Dämpfers zu verstellen, beispielsweise zu verriegeln oder zu öffnen, um die Dämpfung zu verändern. Die Dämpfung wird bevorzugt motorisch verändert, dazu ist die Verstelleinrichtung mit einem Motor ausgestattet, die über eine Steuereinrichtung und eine Sensoranordnung während des Gehens Daten bereitstellt und mit einem Prozessor innerhalb der Steuereinrichtung verarbeitet. Basierend auf den Sensordaten wird dann von der Steuereinrichtung, die zudem ein entsprechendes Datenverarbeitungsprogramm, einen Speicher und eine Energieversorgung aufweisen kann, ein Steuersignal an den Motor gesendet, um eine entsprechende Verstellung vorzunehmen.

Der Dämpfer kann sperrbar ausgebildet sein, beispielsweise um nach einer Belastung oder nach einem definierten Einsinken oder Ausfahren des Dämpfers eine einmal gefundene Position zu fixieren, bis eine neue Position eingenommen werden soll. Dies kann durch Öffnen und Schließen von Ventilen oder durch eine mechanische Sperreinrichtung geschehen.

Eine Weiterbildung der Erfindung sieht vor, dass dem Dämpfer ein Folgeventil zugeordnet ist oder dass der Dämpfer ein Folgeventil beinhaltet, das erst bei Überschreiten einer vorgegebenen Kraft oder eines vorbestimmten Momentes öffnet und eine Bewegung des Dämpfers ermöglicht. Die Auslösekraft oder das Auslösemoment können durch eine Steuerung motorisch oder manuell einstellbar sein. Die Steuerung weist neben einer Datenverarbeitungseinrichtung, einem Daten- und gegebenenfalls Energiespeicher einen Aktuator zur Verstellung des Folgeventils auf. Basierend auf Sensordaten, die in dem Prozessor der Steuerungseinrichtung verarbeitet werden, kann das Auslösemoment verstellt werden.

Eine Weiterbildung der Erfindung sieht vor, dass der Halter verstellbar ausgebildet ist, um einen Proximal-Distal-Abstand der Befestigungseinrichtung zu der Hauptfeder einzustellen. Über den Halter ist es möglich, den einstellbaren Proximal-Distal-Abstand zu verändern, wobei der Halter bevorzugt mehrteilig ausgebildet ist und eine Arretiereinrichtung aufweist, über die der Halter in der jeweiligen Position fixierbar ist. Beispielsweise kann der Halter zweiteilig mit zueinander verschwenkbaren Schenkeln aufgebaut sein, wobei an dem oberen, proximalen Schenkel die Befestigungseinrichtung angeordnet oder befestigt ist. Durch die einstellbare Verlagerung ist es möglich, eine Anpassung an unterschiedliche Absatzhöhen vorzunehmen oder den Gesamtprothesenaufbau durch eine Veränderung der Orientierung der Befestigungseinrichtung und damit der proximalen Komponente zu verändern.

In einer Weiterbildung der Erfindung ist es vorgesehen, dass die Befestigungseinrichtung verschieblich, gelenkig oder drehbar an dem Halter gelagert ist. Durch die verschiebliche, gelenkige oder drehbare Lagerung der Befestigungseinrichtung in dem oder in dem Halter ist es möglich, eine Anpassung an unterschiedliche Absatzhöhen oder Ausrichtungen vorzunehmen, bevorzugt ist die Befestigungseinrichtung in der jeweiligen Position relativ zu dem Halter fixierbar, beispielsweise über Klemmschrauben, Stifte, Verzahnungen oder dergleichen.

Nachfolgend werden Ausführungsbeispiele der Erfindung anhand der beigefügten Figuren näher erläutert. Es zeigt:
- Figur 1 -: eine schematische Darstellung eines Prothesenfußeinsatzes;
- Figur 2 -: drei Belastungsphasen des Prothesenfußeinsatzes in einer Fußhülle;
- Figur 3 -: eine Seitenansicht eines unbelasteten Prothesenfußeinsatzes;
- Figur 4 -: eine Schnittdarstellung eines Prothesenfußeinsatzes;
- Figur 5 -: einen Prothesenfußeinsatz bei einem Fersenstoß;
- Figur 6 -: einen Prothesenfußeinsatz in der mittleren Standphase;
- Figur 7 -: Prothesenfußeinsatz beim Überrollen;

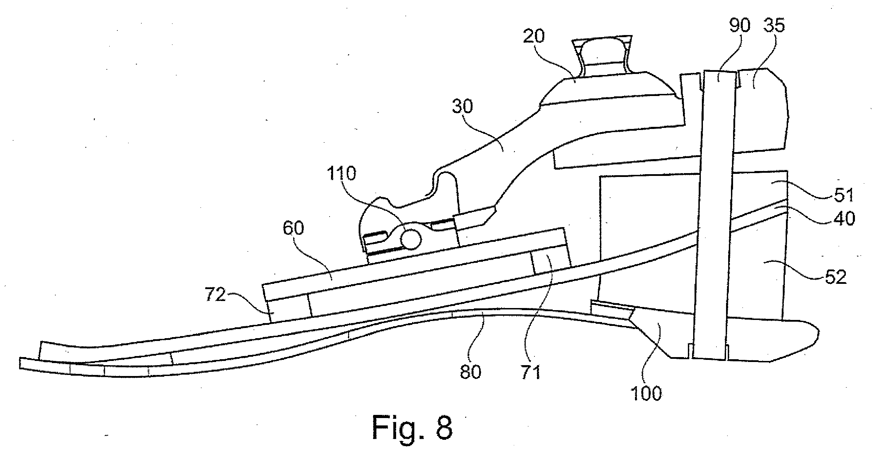

- Figur 8 -: Prothesenfußeinsatz in der terminalen Standphase;
- Figur 9 -: eine Variante der Figur 4 mit einem Dämpfer;
- Figur 10 -: eine Variante der Figur 4 mit einem verstellbaren Halter; sowie
- Figur 11: - eine Variante mit einer verlagerbaren Befestigungseinrichtung
- Figur 12: - eine perspektivische Ansicht eines Prothesenfußeinsatzes;
- Figur 13: eine Explosionsdarstellung des Prothesenfußeinsatzes gemäß Figur 12; sowie
- Figur 14: einen Prothesenfußeinsatz gemäß Figur 12 mit Kosmetik.

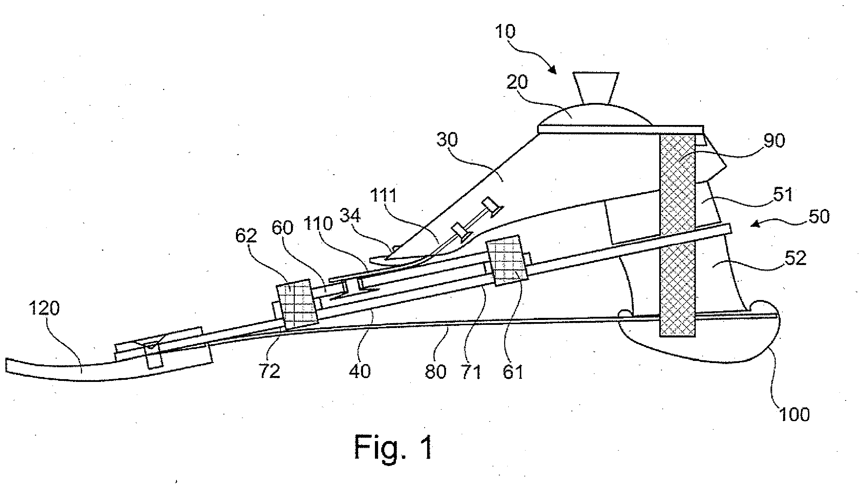

Figur 1 zeigt in einer schematischen Darstellung einen Prothesenfußeinsatz 10 mit einer proximalen Befestigungseinrichtung 20, die beispielsweise als Pyramidenadapter ausgebildet sein kann. Die proximale Befestigungseinrichtung 20 kann einstückig mit einem Halter 30 ausgebildet sein, alternativ kann die Befestigungseinrichtung 20 lösbar an dem Halter 30 befestigt sein. Der Halter 30 besteht aus einem formstabilen Material, beispielsweise einem Leichtmetall, einem Verbundwerkstoff aus einem Kunststoff mit darin eingebetteten Fasern, aus einem Kunststoff oder einem anderen geeigneten Material, um Kräfte und/oder Momente, die von einer nicht dargestellten Proximalkomponente auf den Prothesenfußeinsatz 10 über die proximale Befestigungseinrichtung 20 eingeleitet werden, aufzunehmen und zu verteilen. Der Halter 30 erstreckt sich von der proximalen Befestigungseinrichtung 20 in distale Richtung, also nach unten, und in anteriore Richtung, also in Gangrichtung nach vorn. An dem anterioren, vorderen Ende des Halters 30, der einen schräg nach distal anterior gerichteten Abschnitt aufweist, ist in dem dargestellten Ausführungsbeispiel ein Vorsprung 34 angeordnet oder ausgebildet, der in anteriorer Richtung über das Befestigungselement 20 hinausragt. Ebenfalls ist in dem dargestellten Ausführungsbeispiel an dem Halter 30 ein Träger 111 in Gestalt einer Blattfeder 111 über zwei Schrauben befestigt, die über das anteriore Ende des Halters 30 hinausragt. Die Blattfeder 111 ist über eine weitere Schraube mit einer Zwischenfeder 60 gekoppelt und bildet ein Gelenk 110 zwischen der Zwischenfeder 60 und dem Halter 30 aus, sodass eine Abroll- oder Drehbewegung des Halters 30 mit dem vorderen Ende 34, das abgerundet ist, auf der Blattfeder 111 und damit auf der Zwischenfeder 60 stattfinden kann.

Die Zwischenfeder 60 ist über zwei Blöcke oder Zwischenstücke beabstandet zu einer Hauptfeder 40 in zwei in Längserstreckung der Hauptfeder 40 zueinander beabstandeten Bereichen 71, 72 auf ihr abgestützt. Die Zwischenfeder 60 kann sich auch grundsätzlich direkt auf der Hauptfeder 40 in zwei zueinander beabstandeten Bereichen 71, 72 auf der Hauptfeder 40 abstützen, beispielsweise wenn die Zwischenfeder 60 einen Bogen ausbildet. Der Halter 30 kann auch auf eine andere Art und Weise gelenkig oder verschwenkbar mit der Zwischenfeder 60 verbunden sein.

In dem dargestellten Ausführungsbeispiel ist die Zwischenfeder 60 zusammen mit den Zwischenstücken über Gurte oder Klemmen 61, 62 an der Hauptfeder 40 befestigt. Alternativ zu einer Befestigung über Gurte oder Klemmen 61, 62, die die Zwischenfeder 60, die Zwischenstücke und die Hauptfeder 40 umfänglich umgeben, kann eine Befestigung der Zwischenfeder 60 an der Hauptfeder über eine Schraubverbindung oder eine Klebverbindung stattfinden. Vorteilhafterweise ist die Zwischenfeder 60 reversibel an der Hauptfeder 40 festgelegt, gegebenenfalls über die Zwischenstücke, um eine Anpassung an den jeweiligen Patienten oder geänderte Einsatzzwecke vornehmen zu können.

Die Hauptfeder 40 erstreckt sich von dem hinteren oder posterioren Ende des Halters 30 bis in einen Vorderfußbereich des Prothesenfußeinsatzes 10 und kann sich bis zum anterioren Ende erstrecken. In dem dargestellten Ausführungsbeispiel ist an dem anterioren Ende der Hauptfeder 40 eine separate Zehenfeder 120 mit einer gewölbten Kontur angeordnet, die reversibel über eine Verschraubung an der Hauptfeder 40 festgelegt ist. Über die auswechselbare Zehenfeder 120 kann eine Anpassung an unterschiedliche Schuhgrößen oder Fußgrößen sowie eine Anpassung an die jeweiligen Patientenbedürfnisse hinsichtlich des Abrollverhaltens, insbesondere in der terminalen Standphase, erfolgen. Über eine steife Ausgestaltung der Zehenfeder 120 ist es möglich, die effektive Fußlänge des Prothesenfußeinsatzes 10 zu vergrößern, wird die Zehenfeder 120 vergleichsweise weich gewählt, verkürzt sich die effektive Fußlänge des Prothesenfußeinsatzes 10. Neben einer Schraubverbindung, wie sie in der Figur 1 dargestellt ist, kann die Zehenfeder 120 über eine Steckverbindung, eine Klipsverbindung, eine Klebeverbindung oder eine Verbindung über andere Rastelemente an der Hauptfeder 40 festgelegt werden. Grundsätzlich kann anstatt einer Zehenfeder 120 ein starres oder im Wesentlichen starres Zehenelement eingesetzt und an der Hauptfeder 40 befestigt sein. Das Zehenelement 120 kann klappbar an der Hauptfeder 40 angeordnet sein, beispielsweise über ein Scharnier.

Distal zu der Hauptfeder 40 ist an dem vorderen Ende der Hauptfeder 40 ein Führungselement 80 befestigt. Die Befestigung kann reversibel erfolgen, beispielsweise gemeinsam mit der Befestigung der Zehenfeder 120. Alternativ kann das Führungselement 80 separat unmittelbar an der Hauptfeder 40 reversibel oder formschlüssig oder Stoffschlüssig, beispielsweise über Verkleben oder Verschweißen, festgelegt sein. In einer weiteren Ausführungsform ist es möglich, das Führungselement 80 über die Zehenfeder 120 an der Hauptfeder 40 festzulegen. In einer weiteren Ausführungsform sind das Führungselement 80 und die Hauptfeder 40 biegesteif miteinander verbunden, so dass das Führungselement 80 auch als Feder genutzt werden kann. Das Führungselement 80 ist im Vergleich zu der Hauptfeder 40 wesentlich dünner, beispielsweise nur halb so dick oder weniger als halb so dick wie die Hauptfeder 40. Das Führungselement 80 kann als Blattfeder ausgebildet sein, ebenso wie die Hauptfeder 40 und die Zwischenfeder 60 als Blattfedern ausgebildet sind. Alle drei in dem dargestellten Ausführungsbeispiel gezeigten Blattfedern sind als gerade Blattfedern ausgebildet, was hinsichtlich der Herstellung, Montage, Lagerung und Haltbarkeit vorteilhaft ist. Das Führungselement 80 hat in der dargestellten Ausführungsform vordringlich nicht die Aufgabe, eine Federwirkung bereitzustellen, vielmehr dient das Führungselement 80 zur medial-lateralen Führung eines Fersenelementes 50, das an dem posterioren oder hinteren Ende des Führungselementes 80 angeordnet ist. Das Führungselement 80 kann eine Verlagerung in Medial-Lateral-Richtung sperren und flexibel oder frei schwenkbar in Proximal-Distal-Richtung ausgebildet sein, also eine Verlagerung des hinteren oder posterioren Endes des Führungselementes 80 ohne oder nur mit geringem Widerstand zulassen. An dem Führungselement 80 kann ein Gelenk ausgebildet sein, beispielsweise im Bereich der Befestigung an der Hauptfeder 40 an dem anterioren Ende. Das Gelenk kann als Filmscharnier oder Scharnier mit einer feststehenden Gelenkachse ausgebildet sein. Die Befestigung der Führungselementes 80 kann auch beispielsweise mittig erfolgen oder an jedem beliebigen Ort. Hauptaufgabe des Führungselementes ist, die Positionierung des Fersenelements 50 sicherzustellen.

An dem posterioren Ende des Führungselementes 80 ist ein Sohlenelement 100 angeordnet, das eine zum Boden weisende abgerundete Form aufweist und ein Auftreten und Abrollen über den hinteren Teil des Führungselementes 80 über das Sohlenelement 100 ermöglicht. Das Sohlenelement 100 kann reversibel an dem Führungselement 80 angeordnet sein. Dazu sind Formschlusselemente vorgesehen, über die das Führungselement 80 an dem Sohlenelement 100 befestigt wird, beispielsweise Vorsprünge, Federlaschen, Schrauben und Gewinde, Bolzen, Schnappverbindungen oder ähnliches. Alternativ oder ergänzend können Führungselement 80 und Sohlenelement 100 stoffschlüssig, beispielsweise über eine Klebverbindung, miteinander gekoppelt sein.

In dem Zwischenraum zwischen der Hauptfeder 40 und dem Führungselement 80 ist eine distale Fersenkomponente 52 angeordnet. Zwischen der Hauptfeder 40 und dem Halter 30 ist eine proximale Fersenkomponente 51 angeordnet. Beide Fersenkomponenten 51, 52 sind Teil eines Fersenelementes 50, das eine elastische Abstützung des Prothesenfußeinsatzes 10 im Fersenbereich ermöglicht. Das elastische Fersenelement 50 besteht in dem dargestellten Ausführungsbeispiel aus den beiden Fersenkomponenten 51, 52, wobei die distale Fersenkomponente 52 zusammen mit dem Sohlenelement 100 kombiniert sein kann oder eine Baugruppe aus den beiden Komponenten ausbildet. Das Sohlenelement 100 kann Teil des Fersenelementes 50 sein und weist zwei in proximaler Richtung weisende Vorsprünge auf, die auch als umlaufender Vorsprung oder Rahmen ausgebildet sein können, wobei der Rahmen so ausgebildet ist, dass die distale Fersenkomponente 52 in diesen Rahmen oder zwischen die beiden Vorsprünge eingesetzt werden kann. Dadurch wird die distale Fersenkomponente 52 auf dem Führungselement 80 oder dem Sohlenelement 100 ausgerichtet und gegen ein Verschieben in anteriorer, posteriorer und gegebenenfalls in Medial-Lateral-Richtung gesichert. Die distale Fersenkomponente 52 ist zwischen der Hauptfeder 40 und dem Führungselement 80 unter einer Vorspannung durch ein Spannelement 90 eingeklemmt. Das Spannelement 90 ist medial und lateral zu der proximalen Fersenkomponente 52 geführt, sodass durch das Spannelement 90 ein seitliches Auswandern der Fersenkomponente 52 in Medial-Lateral-Richtung verhindert wird. In dem dargestellten Ausführungsbeispiel ist das Spannelement 90 als ein zugstarres, flexibles Spannelement 90 in Gestalt eines Gurtes, beispielsweise eines Gewebebandes ausgebildet, das zu einer Schlaufe zusammengeführt ist. Das Spannelement 90 ist an dem Halter 30 angeordnet, in dem dargestellten Ausführungsbeispiel über das proximale Ende des Halters 30 in einer Ausnehmung geführt und über eine Scheibe oder ein Sicherungselement oberhalb des Spannelementes 90 daran gesichert. Alternativ kann das Spannelement 90 medial und lateral an dem Halter 30 über Schrauben, Bolzen, Haken oder ähnliche Befestigungselemente gesichert sein. Bevorzugt ist das Spannelement 90 reversibel an dem Halter 30 festgelegt.

Das Spannelement 90 kann medial und lateral neben der Hauptfeder 40 und dem Führungselement 80 geführt sein, alternativ ist es möglich, dass in der Hauptfeder 40 ein Schlitz ausgebildet ist, durch den das Spannelement 90 in Richtung auf das Führungselement 80 geführt ist. Das Spannelement 90 kann unterhalb des Führungselementes 80 durch das Sohlenelement 100 geführt sein, alternativ kann das Spannelement 90 an dem Führungselement 80 oder dem Sohlenelement 100 befestigt sein, sodass ein Ende des Spannelementes 90 an dem Halter 30 und das andere Ende an dem Führungselement 80 oder dem Sohlenelement 100 befestigt ist. Bevorzugt sind zwei Spannelemente 90 an dem Prothesenfußeinsatz 10 angeordnet, eines medial, das andere lateral.

Das Spannelement 90 hält in dem unbelasteten, dargestellten Zustand gemäß Figur 1 das Fersenelement 50 in einer komprimierten Stellung, die Vorspannung ist durch eine Veränderung der Länge des Spannelementes 90 einstellbar. Über das Spannelement 90 wird zudem die maximale Entfernung des Führungselementes 80 oder des Sohlenelementes 100 von dem Halter 30 definiert. Bei Aufbringung einer Zugkraft auf das Spannelement 90, beispielsweise bei einer Vorderfußbelastung, gibt das Spannelement 90 nicht oder im Wesentlichen nicht nach. Sollte eine Verlängerung des Spannelementes 90 stattfinden, ist nicht beabsichtigt, dass die Verlängerung so groß ist, dass eine Kompression zwischen der Hauptfeder 40 und dem Führungselement 80 auf die distale Fersenkomponente 52 aufgehoben wird. Statt einer Ausgestaltung des Spannelementes 90 als Schlaufe kann dieses auch als zentraler Gurt, als zentrales Seil oder auch als flexibler Stab ausgebildet und in einer Führung an oder durch die Fersenkomponenten 51, 52 gelagert sein, wodurch ebenfalls eine Zuordnung der Bauteile zueinander bereitgestellt und ein mediales und/oder laterales Verlagern verhindert oder vermindert wird.

Die Fersenkomponenten 51, 52 bestehen aus einem elastischen Werkstoff, insbesondere einem elastischen Schaumwerkstoff.

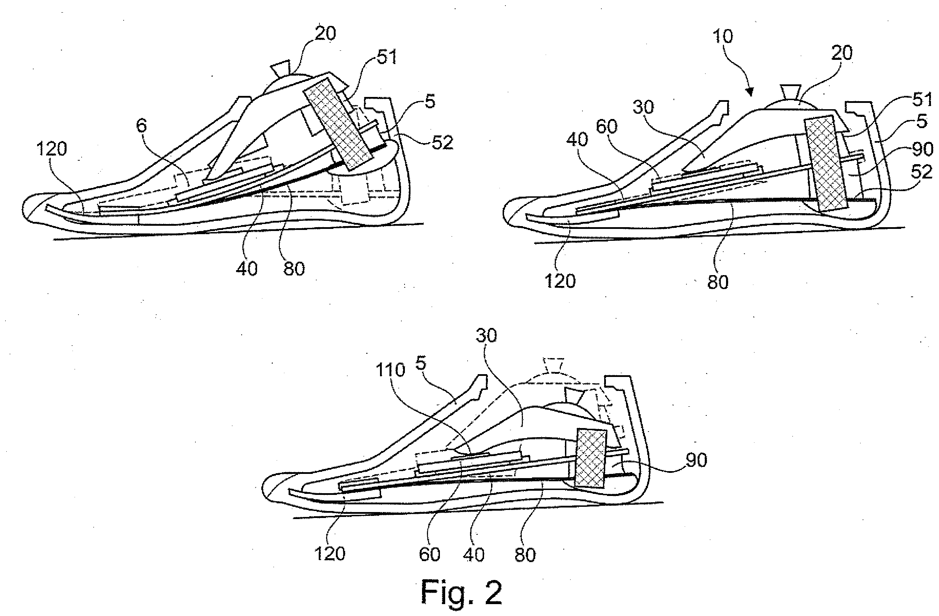

Die Funktionsweise des Prothesenfußeinsatzes wird anhand der Belastungszustände erklärt, die in der Figur 2 dargestellt sind. In der Figur 2 sind drei Belastungszustände eines Prothesenfußeinsatzes 10 in einer Fußhülle 5 dargestellt. Die Darstellung oben rechts der Figur 2 zeigt einen entlasteten Zustand des Prothesenfußeinsatzes 10, die untere Darstellung einen Prothesenfußeinsatz 10 bei einem Fersenauftritt, dem sogenannten heel strike, die obere linke Darstellung den Prothesenfußeinsatz 10 am Ende der Standphase bei einer Vorfußbelastung. Die Fußhülle 5 ist jeweils unverändert in der mittleren Ausgangsposition dargestellt, in der oberen linken und unteren Darstellung sind die Prothesenfußeinsätze 10 in Ausgangsstellung ebenfalls dargestellt. Die obere rechte Darstellung der Figur 2 entspricht der Darstellung gemäß Figur 1, jedoch ohne die Blattfeder 111 oder einen Blechstreifen oder ähnliches als Träger 111 mit dem Gelenk 110 sowie ohne eine detaillierte Darstellung der Befestigung der Zwischenfeder 60 auf der Hauptfeder 40. Die Zwischenfeder 60 kann beispielsweise über Abrollelemente oder Polsterelemente an dem posterioren und anterioren Ende der Zwischenfeder 60 auf der Hauptfeder 40 befestigt, beispielsweise festgeklebt sein. An der Unterseite der Hauptfeder 40 kann ein Polsterelement angeordnet sein, das verhindert, dass eine direkte Anlage des Führungselementes 80 an der Unterseite der Hauptfeder 40 stattfindet, was zu Reibungen und Abnutzungen der in der Regel aus einem Faserverbundwerkstoff bestehenden Blattfedern führen könnte.

In der unteren Darstellung der Figur 2 ist die Belastungssituation bei einem Fersenauftritt oder heel strike gezeigt, bei der der Halter 30 um den Auflagerpunkt auf der Zwischenfeder 60 in Uhrzeigerrichtung verkippt ist. Sowohl die obere, proximale Fersenkomponente 51 als auch die untere, distale Fersenkomponente 52 sind komprimiert, das Spannelement 90 ist entspannt und der Zwischenraum zwischen der Hauptfeder 40 und dem Führungselement 80 ist verkleinert. Das proximale Ende der Hauptfeder 40 hat sich in Richtung auf das Sohlenelement oder zum Boden hin bewegt, ebenso hat sich das posteriore Ende des Halters 30 in Richtung auf die Hauptfeder 40 bewegt, sodass der Halter 30 die Hauptfeder 40 nahezu berührt. Die Belastung bei einem Fersenauftritt erfolgt überwiegend im posterioren Teil über das Fersenelement 50 und das Sohlenelement 100, sodass die Zwischenfeder 60 ebenso wie die Hauptfeder 40 und das Führungselement 80 im Wesentlichen entlastet sind. Gleiches gilt für die Zehenfeder 120, die mit ihrem vorderen Ende in einer Ausnehmung in der Fußhülle 5 eingeführt ist und darin formschlüssig gehalten wird.

Während des Überrollens oder des Stehens, wie es beispielsweise in der oberen rechten Darstellung der Figur 2 gezeigt ist, erfolgt eine gleichmäßige vertikale Belastung über das Fersenelement 50 und den Halter 30 über die Zwischenfeder 60, die Zwischenstücke oder Distanzelemente, die Hauptfeder 40 und die Zehenfeder 120.

In der linken oberen Darstellung ist eine Belastung am Ende der Standphase gezeigt. Das Sohlenelement ist von dem Boden abgehoben, die maximale Belastung findet an dem Berührpunkt der Zehenfeder 120 an dem Boden statt. Die Bodenreaktionskraft wird in die Hauptfeder 40 eingeleitet, der Halter 30 verschwenkt um die Lagerstelle 110 auf der Zwischenfeder 60. Durch die beabstandete Lagerung in den anterioren und posterioren Auflagerbereichen 72, 71 der Zwischenfeder 60 auf der Hauptfeder ist es möglich, die ansonsten über den vorderen Vorsprung 34 auf einem einzelnen Punkt auf der Hauptfeder 40 eingeleiteten Kräfte über den abstützenden Halter 30 auf zwei in Längserstreckung der Hauptfeder 40 zueinander beabstandeten Punkten oder Bereichen zu verteilen, sodass eine gleichmäßige oder vergleichmäßigte Krafteinleitung in die Hauptfeder 40 an zwei Stellen stattfindet. Der Halter 30 wird über das Spannelement 90 mit dem Sohlenelement oder dem Führungselement 80 gekoppelt. Durch die Verdrehung entgegen dem Uhrzeigersinn wird die distale Fersenkomponente 52 durch das Spannelement 90 und das Führungselement 80 gegen die Hauptfeder 40 komprimiert. Die proximale Fersenkomponente, die an dem Halter 30 festgelegt ist, hebt sich von der Hauptfeder 40 ab und wird maximal dekomprimiert. Somit findet eine Biegung zwischen dem vorderen Auflagerpunkt der Hauptfeder 40 und dem Angriffspunkt oder Angriffsbereich der distalen Fersenkomponente 52 der Hauptfeder 40 statt. Anders als bei dem Fersenauftritt oder heel strike, bei dem die Hauptfeder 40 nicht gebogen ist und die gesamte Feder- und Dämpfungswirkung über das Fersenelement 50 mit den beiden Fersenkomponenten 51, 52 und gegebenenfalls über das Sohlenelement in Zusammenwirkung mit der Fußkosmetik 5 stattfindet, wird bei dem Abrollen des Fußes und eine Vorderfußbelastung die Hauptfeder 40 in Aktion gebracht. Sobald der Krafteinleitungsvektor vor das Befestigungselement 20 wandert, wird über das vordere Ende des Halters 30 eine Druckkraft über die Zwischenfeder 60 auf die Hauptfeder 40 aufgebracht, was das Spannelement 90 spannt und eine Biegung sowohl insbesondere der Hauptfeder 40 als auch eine Biegung des Führungselementes 80 und eine Kompression der distalen Fersenkomponente 52 zur Folge hat.

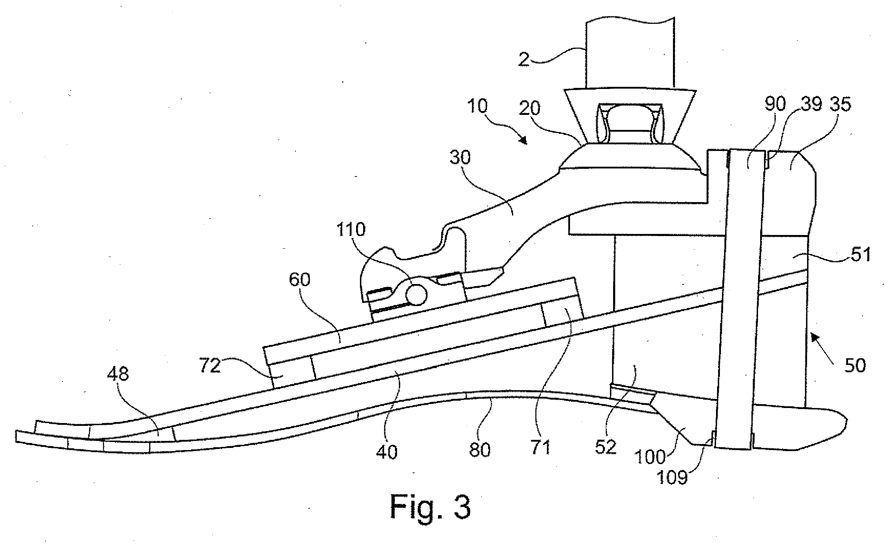

Figur 3 zeigt eine Variante des Prothesenfußeinsatzes 10 gemäß der Figuren 1 oder 2. Der grundsätzliche Aufbau mit dem Befestigungselement 20, dem Halter 30, der sich mit seinem vorderen Ende über eine Zwischenfeder 60 über zwei Zwischenstücke in den Bereichen 71, 72 auf eine Hauptfeder 40 abstützt, die in ihrem vorderen Bereich mit einem Führungselement 80 verbunden ist, das in seinem hinteren oder posterioren Bereich ein Fersenelement 50 führt, das über ein Spannelement 90 auf Kompression gehalten ist, ist gleich geblieben.

In der Figur 3, die eine Seitenansicht des unbelasteten Prothesenfußeinsatzes 10 zeigt, ist zudem die Proximalkomponente 2 in Gestalt eines Unterschenkelrohrs gezeigt, das reversibel an der Befestigungseinrichtung 20 über einen üblichen Pyramidenadapter festlegbar ist. An dem vorderen Ende des Halters 30 ist statt des Bleches 111 oder der Blattfeder 111 als Träger, die an der Zwischenfeder 60 gelenkig gelagert ist, in der Ausführungsform gemäß der Figur 3 das Gelenk 110 mit einer definierten Gelenkachse ausgebildet, die über ein Befestigungselement, beispielsweise ein Klemmadapter an der Zwischenfeder 60 befestigt ist. Die Achse kann an dem vorderen Ende des Halters 30 befestigt oder ausgebildet sein, beispielsweise als Achsstümpfe, die schwenkbar in zwei Aufnahmen medial und lateral zu dem Halter 30 an der Zwischenfeder 60 festgelegt sind.

Die Hauptfeder 40 ist in ihrem anterioren, vorderen Bereich von der Unterseite gesehen konvex gebogen, sodass ein leichteres Abrollen bei einer Vorderfußbelastung erfolgen kann. Das Führungselement 80 ist über eine Verbindungseinrichtung 48, beispielsweise eine Klebeverbindung oder eine formschlüssige Verbindung, mit dem Führungselement 80 verbunden. Das Führungselement 80 weist eine gewellte Form auf, die im Vorderfußbereich konvex geformt ist, im Mittelfußbereich konkav und im Fersenbereich gerade oder ebenfalls konvex, jeweils von unten gesehen. Über den Verlauf von anterior nach posterior verjüngt sich das Führungselement 80 im Mittelfußbereich, kann aber auch eine gleichbleibende Materialstärke aufweisen, die wesentlich geringer als die der Hauptfeder 40 ist.

Das Sohlenelement 100 weist eine Ausnehmung 109 zum Führen des Spannelementes 90 auf. Korrespondierend ist eine Ausnehmung 39 in einem Fersenteilhalter 35 ausgebildet, in der das Spannelement 90 geführt ist, sodass keine Bewegung in anterior-posteriorer Richtung im entlasteten Zustand möglich ist. Der Fersenteilhalter 35 kann auswechselbar an dem Halter 30 angeordnet sein, beispielsweise aufsteckbar und durch eine formschlüssige Schnappverbindung verriegelbar ausgebildet sein. Ebenso ist es möglich, dass der Fersenteilhalter 35 dauerhaft und irreversibel an dem Halter 30 festgelegt ist, beispielsweise angeschweißt oder angeklebt. Der Ferseneilhalter 35 ist Teil des Halters 30.

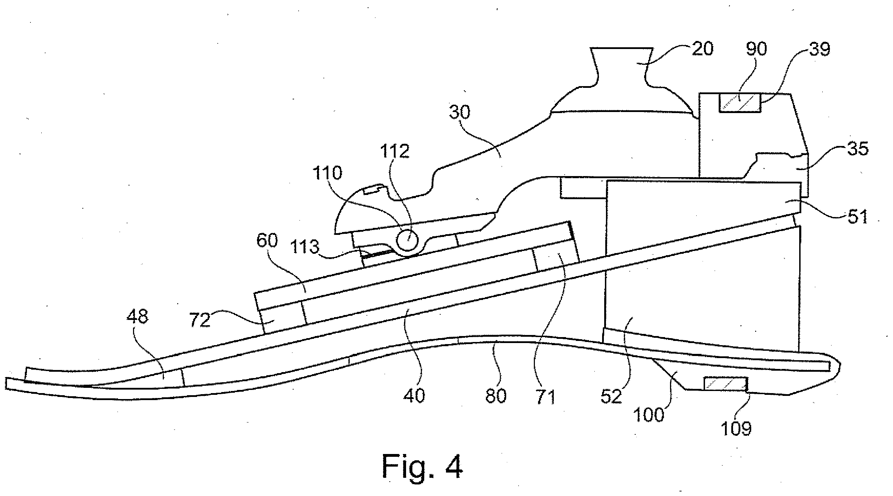

Figur 4 zeigt den Prothesenfußeinsatz 10 gemäß Figur 3 in einer Schnittdarstellung, die eckige Kontur der Ausnehmungen 39, 109 zur Aufnahme des gurtartigen, zugstarren aber flexiblen Spannelementes 90 sind ebenso zu erkennen wie die im Wesentlichen geradlinige Ausgestaltung der Hauptfeder 40 und der Zwischenfeder 60. Das Gelenk 110 an dem vorderen Ende des Halters 30 weist einen Achsbolzen 112 auf, der schwenkbar an einer Achsbolzenaufnahme 113 aufgenommen ist. Die Zwischenstücke zur beabstandeten Festlegung der Zwischenfeder 60 an der Hauptfeder 40 sind in dem dargestellten Ausführungsbeispiel stoffschlüssig über eine Verklebung mit der Zwischenfeder 60 und der Hauptfeder 40 verbunden.

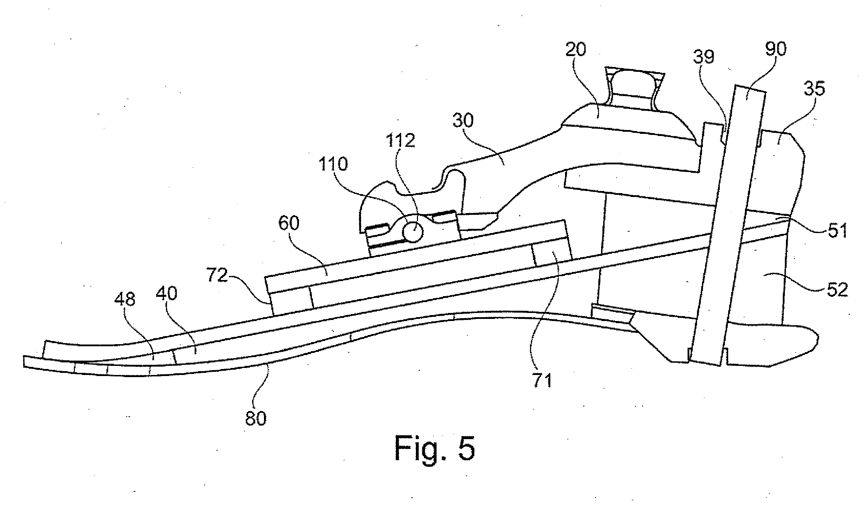

Figur 5 zeigt den Prothesenfußeinsatz gemäß Figur 3 während des Fersenauftrittes oder während des heel strikes. Die proximale Fersenkomponente 51 ist maximal komprimiert, der Fersenteilhalter 35 an dem posterioren Ende des Halters 30 komprimiert den insbesondere posterioren Teil der Fersenkomponente 51, ebenso wie die distale Fersenkomponente 52. Der Halter 30 ist um den Bolzen 112 im Uhrzeigersinn verschwenkt, das Spannelement 30 ist in dem dargestellten Ausführungsbeispiel ausreichend starr, sodass es sich aus der Ausnehmung 39 herausbewegt. Sofern das Spannelement 90 in dem Bereich der Ausnehmung 39 festgelegt ist, beispielsweise angeschraubt, eingeklemmt oder verklebt, wird sich das Spannelement 90 bei einer Kompression aufgrund eines Fersenauftrittes medial und lateral nach außen bewegen. Eine Biegung der Hauptfeder 40 findet nicht statt. Eine Annäherung des Führungselementes 80 an die Unterseite der Hauptfeder findet im Mittelfußbereich aufgrund der Kompression der distalen Fersenkomponente 52 statt.

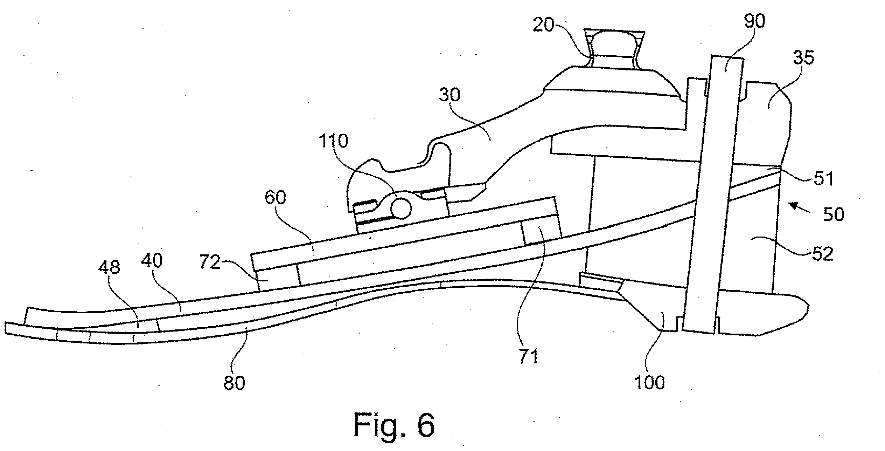

Figur 6 zeigt eine fortschreitende Bewegung nach dem Fersenauftritt gemäß Figur 5, bei der der Vorderfußbereich abgesenkt ist und die nicht mehr dargestellte Proximalkomponente 2 der Prothese entgegen dem Uhrzeigersinn in Gangrichtung nach vorne verschwenkt wird. Dadurch wird die Druckkraft auf das anteriore Ende des Halters 30 vergrößert, sodass über das Gelenk 110 eine Druckkraft zentral auf die Zwischenfeder 60 ausgeübt wird, die dann über die beiden Lagerblöcke die Druckkraft in Längsrichtung beabstandet aufgeteilt in die Hauptfeder 40 einleitet. Die Hauptfeder 40 wird dadurch zwischen dem vorderen Auflagepunkt und dem hinteren Ende durchgebogen und nähert sich im Mittefußbereich dem Führungselement 80 an. Das Spannelement 90 bewegt sich wieder in Richtung auf den Fersenteilhalter 35 bei zunehmender Entlastung des elastischen Fersenelementes 50.

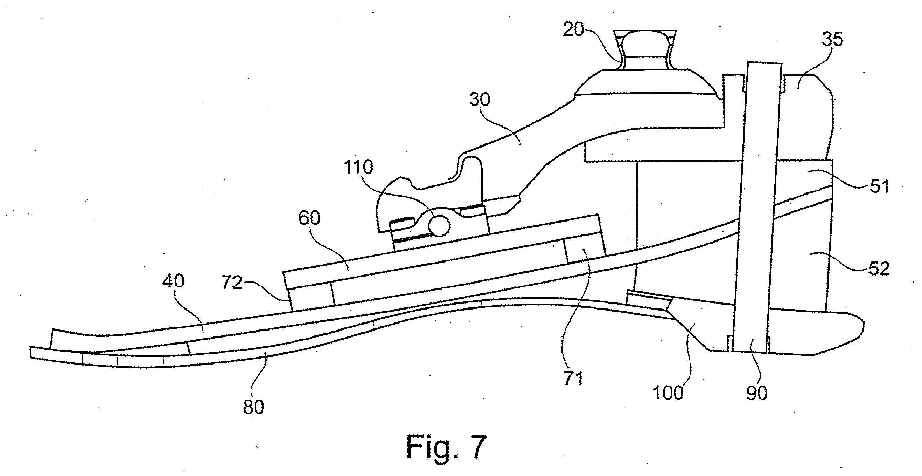

Figur 7 zeigt den Prothesenfußeinsatz 10 bei zunehmender Vorfußbelastung, der Krafteinleitungspunkt ist weiter nach vorne gewandert, die beiden Fersenkomponenten 51. 52 sind nahezu vollständig entlastet. Die proximale Fersenkomponente 51 liegt mit ihrer Oberseite an der Unterseite des Halters 30 bzw. des Fersenteilhalters 35 an, über das Spannelement 90 wird eine Zugkraft von dem Halter 30 über den Fersenteilhalter 35 auf das Sohlenelement 100 und damit auf das Führungselement 80 übertragen. Dadurch wird die distale Fersenkomponente 52 komprimiert und gegen die Hauptfeder 40 gedrückt, so dass neben der Durchbiegung der Hauptfeder 40 durch die beabstandete Einleitung von Druckkräften in den beiden Bereichen 71, 72 eine Rückstellkraft über die elastische distale Fersenkomponente 52 bereitgestellt wird. Zusätzlich zu den Federeigenschaften der Zwischenfeder 60 und der Hauptfeder 40 wird über die distale Fersenkomponente 52 eine weitere elastische Komponente bereitgestellt, über die es zusätzlich möglich ist, die Federeigenschaften und Rückfedereigenschaften des Prothesenfußeinsatzes 10 einzustellen.

Bei einer weiteren Abrollbewegung des Prothesenfußes wird die Vorfußlast weiter erhöht, das Führungselement 80 nähert sich weiter der Unterseite der Hauptfeder 40 an und die distale Fersenkomponente 52 wird weiter komprimiert. In dem dargestellten Ausführungsbeispiel ist die proximale Fersenkomponente 51 an der Hauptfeder 40 festgelegt, sodass sich der Halter 30 oder, bei einem vorhandenen Fersenteilhalter 35, der Fersenteilhafter 35 von der proximalen Fersenkomponente 51 ablöst. Die proximale Fersenkomponente 51 hat bei einer Vorfußbelastung, wenn der Krafteinleitungsvektor von der proximalen Komponente 2 zu einer Verschwenkung entgegen dem Uhrzeigersinn um das Gelenk 110 führt, keine Federwirkung mehr.

Durch die unterschiedlichen Belastungssituationen der jeweiligen Fersenkomponenten 51, 52 ist es möglich, die Federeigenschaften des Prothesenfußeinsatzes 10 individuell anzupassen. So kann beispielsweise die distale Fersenkomponente 52 härter als die proximale Fersenkomponente 51 ausgebildet sein. Eine härtere distale Fersenkomponente 52 bedeutet, dass ein Widerstand gegen eine Verformung größer als der einer proximalen Fersenkomponente 51 bereitgestellt wird. Dadurch ist es möglich, einen zunächst weichen Fersenauftritt für den Patienten über die proximale Fersenkomponente 51 zu ermöglichen. Darüber hinaus ist es möglich, den weichen Einfederweg über die Dimension der proximalen Fersenkomponente 51 festzulegen. Ab einer bestimmten Kompression und Verformung der proximalen Fersenkomponente 51 wirkt zusätzlich die distale Fersenkomponente 52. Aufgrund der größeren Federsteifigkeit der distalen Fersenkomponente 52 führt dies bei deren Wirksamwerdung zu einer schnellen Plantarflexion und einem sicheren Stand. Die distale Fersenkomponente 52 gibt bei einer zusätzlichen Fersenlast weiter nach, wenn auch in einem weniger großen Umfang verglichen mit der distalen Fersenkomponente 51.

Die Abstützung der bei einer Vorfußlast von dem Halter 30 auf die Hauptfeder 40 übertragenden Kräfte an zwei zueinander beabstandeten Stellen oder Bereichen 71, 72 ermöglicht eine gleichmäßige Krafteinleitung, so dass die Federeigenschaften der als Blattfeder ausgebildeten Hauptfeder 40 optimal ausgenutzt werden können. Dadurch kann die Hauptfeder 40 vergleichsweise dünner und leichter ausgebildet sein oder aber bei einer gleichen Ausgestaltung eine höhere Haltbarkeit aufweisen, verglichen mit einer punktuellen Krafteinleitung. Die Zwischenfeder 60 kann auswechselbar ausgebildet sein und an den jeweiligen Patienten oder die vorgesehenen Belastungen angepasst werden.

Durch die nur einseitige Befestigung der proximale Fersenkomponente 51 entweder an dem Halter 30 oder an der Hauptfeder 40 werden keine Zugkräfte durch die proximale Fersenkomponente 51 übertragen, was der Haltbarkeit der vorzugsweise aus einem Schaummaterial bestehenden proximalen Fersenkomponente 51 dient. Die distale Fersenkomponente 52 ist bevorzugt permanent in einem vorgespannten Zustand zwischen der Hauptfeder 40 und dem Führungselement 80 oder dem Sohlenelement 100.

Das Gelenk 110 ist bevorzugt im Mittelfußbereich, insbesondere bevorzugt in der Mitte der Hauptfeder 40 angeordnet, sodass sich eine optimale Kraftverteilung von dem Halter 30 auf die Hauptfeder 40 erzielen lässt, wenn die Bereiche 71, 72 gleichmäßig in Längserstreckung des Prothesenfußeinsatzes 10 zu dem Gelenk 110 angeordnet sind. Durch eine Verschiebung der Bereiche 71, 72 oder eine ungleichmäßige Beabstandung von dem Gelenk 110 können unterschiedliche Federeigenschaften der Hauptfeder 40 realisiert werden.

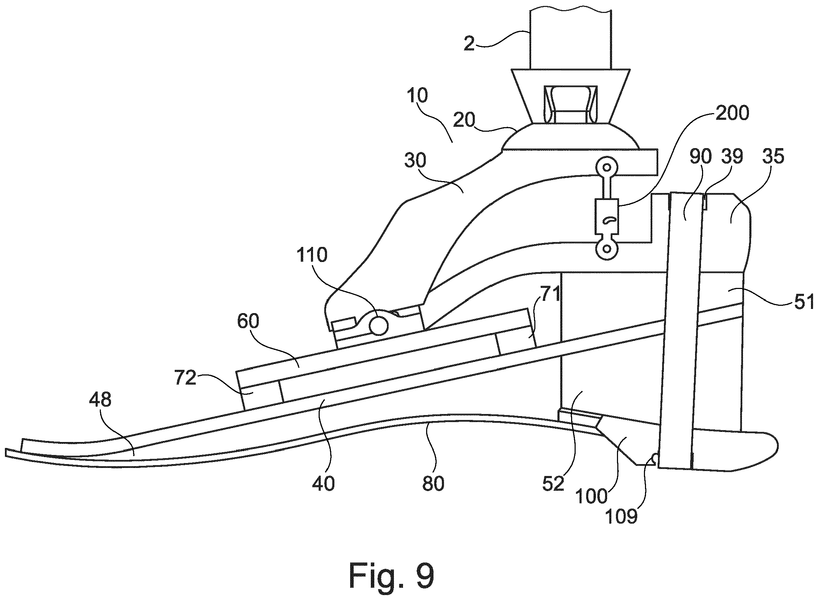

In der Figur 9 ist eine Variante der Figur 4 gezeigt, bei der zwischen der Hauptfeder 4 und dem Halter 30 ein Dämpfer 200 angeordnet ist. Im dargestellten Ausführungsbeispiel ist der Dämpfer 200 zwischen einem proximalen, oberen Schenkel des Halters 30 und einem unteren Schenkel, der sich auf der Zwischenfeder 60 und der proximalen Fersenkomponente 51 auf der Hauptfeder 40 abstützt, gelagert. Der distale Schenkel ist schwenkbar um die Schwenkachse des Gelenkes 110 gelagert, sodass die Befestigungseinrichtung 20 und damit auch die Proximalkomponente 2 relativ zu der Hauptfeder 40 verschwenkbar ist. Der Dämpfer 200 kann als Pneumatikdämpfer und/oder Hydraulikdämpfer ausgebildet sein, alternativ kann der Dämpfer 200 als Aktuator ausgebildet sein, der in einem Antriebsmodus und/oder in einem Dämpfungsmodus betreibbar ist. Bei einer Ausgestaltung als Aktuator kann eine aktive Veränderung des Abstandes zwischen den beiden Schenkeln und damit des Abstandes zwischen der Befestigungseinrichtung 20 und der Hauptfeder 40 erfolgen. Über den Aktuator kann eine motorische Verstellung hinsichtlich der Neigung aufgrund der Verschwenkung des proximalen Schenkels und der daran angeordneten Befestigungseinrichtung 20 relativ zu der Hauptfeder 40 erfolgen, wodurch beispielsweise eine Anpassung an unterschiedliche Absatzhöhen vorgenommen werden kann. Bei einer Ausgestaltung als reiner Dämpfer kann beispielsweise eine konstante Kraft oder ein konstantes Moment um das Gelenk 110 dazu führen, dass eine Absenkung oder eine Vorwärtsverkippung erfolgt. Ein langsames Einsinken oder Anheben ermöglicht eine korrekte Einstellung der gewünschten Absatzhöhe, beispielsweise indem Ventile innerhalb des Dämpfers 200 geschlossen werden, wenn die gewünschte Stellung erreicht ist. Ebenso kann bei einer Ausgestaltung als Aktuator ein Antrieb gestoppt und verriegelt werden, wenn die gewünschte Orientierung bzw. der gewünschte Abstand und/oder Winkel der Befestigungseinrichtung 20 relativ zu der Hauptfeder 40 bzw. dem Boden erreicht ist. Grundsätzlich ist es auch möglich, die Dämpfung motorisch zu verändern, wenn ein Aktuator oder Motor hierfür vorgesehen ist. Der Motor oder Antrieb kann dann über eine Steuereinrichtung und eine Sensoranordnung während des Gehens verstellt werden, um eine Anpassung an unterschiedliche Gehgeschwindigkeiten, Belastungen oder Gehsituationen zu ermöglichen. Bei einer Ausgestaltung des Dämpfers 200 als Hydraulik- und/oder Pneumatikdämpfer kann dieser mit einem Folgeventil ausgestattet sein, das erst bei Überschreitung eines vorgegebenen Momentes oder einer vorgegebenen Kraft die Bewegung freigibt und somit als eine Art Überlastschutz wirkt. Darüber hinaus kann über das Folgeventil bei Überschreiten der Grenzkraft eine Verstellung durchgeführt und nach Wegfall der entsprechend hohen Verstellkraft eine Verriegelung in der gewünschten Position beibehalten werden.

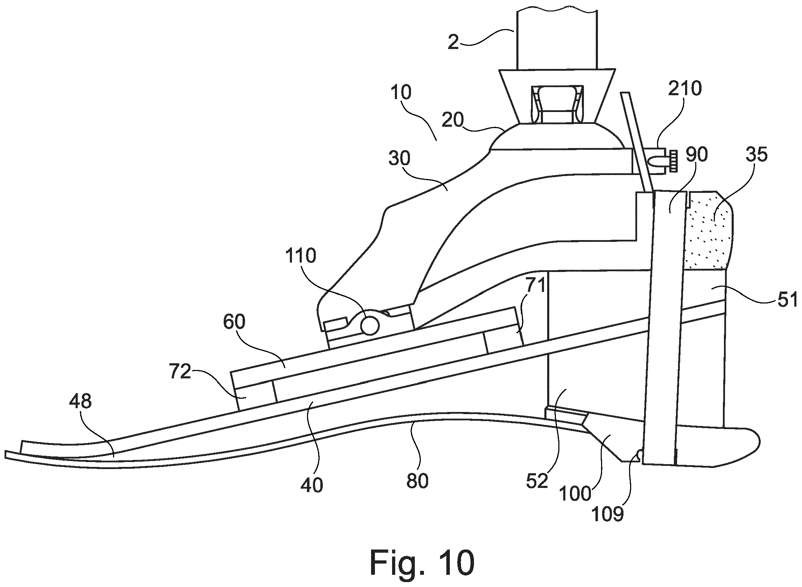

Figur 10 zeigt eine weitere Variante des Prothesenfußeinsatzes, dessen konstruktiver Aufbau im Wesentlichen dem der Figur 9 entspricht. Auch hier ist der Halter 30 zweigeteilt und weist einen proximalen Schenkel auf, der um das Gelenk 110 und eine Schwenkachse relativ zu einem distalen Schenkel verlagerbar ist. In dem posterioren Bereich des proximalen Schenkels ist eine Arretiereinrichtung 210 angeordnet, über die der proximale Schenkel in diskreten Schritten oder stufenlos relativ zu dem distalen Schenkel verstellt und in der jeweiligen Position fixiert werden kann. Die Verstellung der beiden Schenkel zueinander kann auch motorisch erfolgen. Der Motor oder Antrieb kann dann über eine Steuereinrichtung oder eine Sensoranordnung während des Gehens aktiviert und deaktiviert werden.

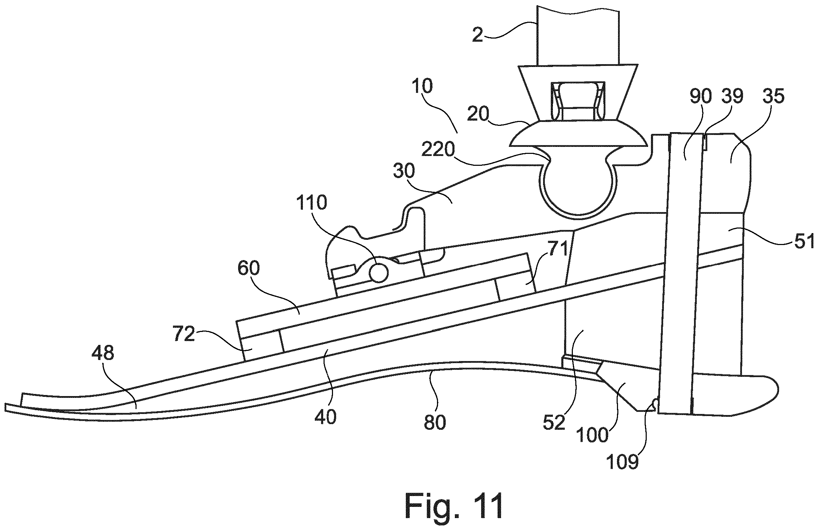

Figur 11 zeigt eine weitere Variante des Prothesenfußeinsatzes mit einer verlagerbar an dem Halter 30 angeordneten Befestigungseinrichtung 20. Die Befestigungseinrichtung 20 weist beispielsweise eine distale Kugelkomponente 220 auf, die in eine korrespondierende Ausnehmung innerhalb des Halters 30 eingebracht ist. Andere Lagerungskonzepte wie Kardangelenk oder eine einachsige oder mehrachsige Verschwenkbarkeit sind Alternativen. Über die Lagerung ist ein Verschwenken, Verdrehen oder Verschieben der Befestigungseinrichtung 20 relativ zu dem Halter 30 gegeben. Ist die Zielposition der Befestigungseinrichtung 20 erreicht, beispielsweise im Rahmen einer Absatzhöhenanpassung, wird die Befestigungseinrichtung 20 an dem Halter 30 fixiert, beispielsweise durch Klemmelemente, Schrauben, Formschlusselemente oder durch Unterbrechung eines Antriebes einer selbsthemmenden Antriebseinrichtung, beispielsweise einer Spindel. Die verlagerbare Befestigungseinrichtung 20 gemäß Figur 11 kann auch bei einer mehrteiligen Ausgestaltung des Halters 30 gemäß der Figuren 9 und 10 vorgesehen sein, insbesondere wenn eine Absatzhöhenanpassung durch eine Veränderung des Distal-Proximal-Abstandes der Befestigungseinrichtung 20 relativ zu der Hauptfeder 40 erfolgt. Neben der Arretiereinrichtung 210, die in der Figur 10 dargestellt ist, können andere Arretiereinrichtungen oder Fixierelemente vorgesehen sein, um die Position des Halters 30 und die Position der Befestigungseinrichtung 20 relativ zu dem Halter 30 zu fixieren, beispielsweise durch Distanzelemente, Anschläge, Stellschrauben, Klemmschrauben oder dergleichen.

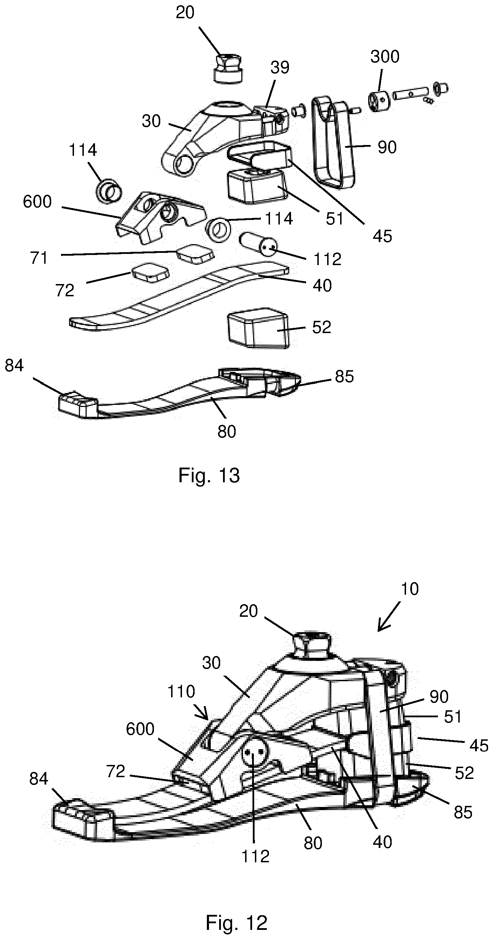

In der Figur 12 ist in einer perspektivischen Gesamtdarstellung ein Prothesenfußeinsatz 10 einer weiteren Variante der Erfindung gezeigt. Das Befestigungselement 20 an dem Halter 30 ist als Pyramidenadapter ausgebildet und dient zur Befestigung an einer nicht dargestellten, proximalen Prothesenkomponente, beispielsweise einem Unterschenkelrohr oder einem Unterschenkelschaft. Der Halter 30 ist insbesondere aus einem formstabilen Material ausgebildet und ist an seinem vorderen Ende mit einer Bohrung versehen, durch die ein Achsbolzen 112 durchgeführt ist, um ein Gelenk 110 zwischen dem Halter 30 und einem Träger 600 zu bilden. Der Träger 600 entspricht der Zwischenfehler oder dem Zwischenelement der vorherigen Ausführungsformen, ohne jedoch signifikante Federungseigenschaften aufzuweisen. An dem vorderen und hinteren Ende des Trägers 600 sind seitliche Vorsprünge angeordnet, die im Wesentlichen in der Breite der Hauptfeder 40 zueinander beabstandet sind, sodass die Hauptfeder 40 zwischen den Vorsprüngen aufgenommen werden kann. Der Träger 600 stützt sich über Zwischenstücke, von denen in dieser Darstellung nur das vordere Zwischenstück 72 zu erkennen ist, auf der Hauptfeder 40 ab. Die Hauptfeder 40 kann über eine Klebeverbindung mit dem Träger 600 verbunden sein. Ebenso ist es möglich, dass der Träger Quervorsprünge an dem unteren Ende der Vorsprünge aufweist, sodass eine C-förmige oder schlitzartige Aufnahme für die Hauptfeder 40 ausgebildet wird. Die Hauptfeder 40 kann dann in diese Aufnahme eingeschoben werden. Eine Fixierung der Hauptfeder 40 an dem Träger 600 kann dann über die Zwischenstücke, Formschlusselemente, eine Klemmverbindung und/oder eine Klebeverbindung erfolgen.

Das vordere Ende der Hauptfeder 40 ist formschlüssig an dem Führungselement 80 oder einer Basisfeder gelagert. Dazu ist an dem vorderen Ende des Führungselementes 80 eine vordere Aufnahme 84 oder eine Tasche ausgebildet, in die die Hauptfeder 40 eingeführt ist. Eine zusätzliche Fixierung kann über Stifte, Bolzen, Haken, Schrauben, Clipsverbindungen, Klettverschlüsse, über andere formschlüssigen Verbindungen und/oder klemmende Verbindungen und/oder stoffschlüssige Verbindungen erfolgen. Bevorzugt ist die Hauptfeder 40 an dem vorderen Ende des Führungselementes 80 lösbar und auswechselbar gelagert, sodass eine zerstörungsfreie Entnahme der Hauptfeder 40 oder ein Auswechseln des Führungselementes 80 zu Reparaturzwecken, zu Anpassungszwecken oder zu Einstellungszwecken erfolgen kann. Die vordere Aufnahme 84 kann als integraler Bestandteil des Führungselementes 80 ausgebildet sein. Alternativ kann die vordere Aufnahme 84 als separates Element gefertigt und dauerhaft an dem Führungselement 80 befestigt sein, beispielsweise angeschweißt, angeklebt oder über Befestigungselemente fixiert.

An dem hinteren Ende des Führungselementes 80 ist eine hintere Aufnahme 85 für das Fersenelement 50 angeordnet oder ausgebildet. Auch die hintere Aufnahme 85 kann entweder einstückig als integraler Bestandteil des Führungselementes 80 ausgebildet oder separat gefertigt und an dem Führungselementes 80 befestigt sein, entsprechend zu der vorderen Aufnahme 84. Die hintere Aufnahme 85 weist allseitig vorstehende Bereiche auf, die in proximalen Richtung orientiert sind, sodass innerhalb dieser vorstehenden Bereiche die distale Fersenkomponente 52 eingesetzt werden kann. Über die hintere Aufnahme 85 wird gewährleistet, dass die distale Fersenkomponente 52 keine seitlichen oder in und entgegen der Gehrichtung gerichteten Bewegungen ausführen kann. Ebenfalls ist eine Verdrehung der distalen Fersenkomponente 52 nicht möglich und die distale Fersenkomponente 52 ist auf dem Führungselement 80 aufgesetzt.

Oberhalb der distalen Fersenkomponente 52 befindet sich die Hauptfeder 40, auf deren hinteren Ende eine Kappe 45 aufgeschoben oder aufgesetzt ist, die einen seitlichen und hinteren, rahmenartigen Vorsprung ausgebildet. Dadurch wird verhindert, dass das obere Ende der distalen Fersenkomponente 52 sich seitlich oder nach hinten verschieben kann. Der seitliche und hintere Vorsprung der Kappe 45 erstreckt sich auch in proximaler Richtung von der Oberseite der Hauptfeder 40 weg und dient damit als Aufnahme und Führung für die proximale Fersenkomponente 51. Die proximale Fersenkomponente 51 liegt mit ihrer proximalen Oberseite auf der Unterseite des Halters 30 auf, entweder unmittelbar oder über Zwischenstücke oder einen weiteren Halter.

Als Spannelement 90 ist ein Gurt über die Oberseite des Halters 50 und medial und lateral der beiden Fersenkomponenten 51, 52 der Hauptfeder 40 geführt. An der Unterseite ist der Gurt 90 unterhalb der hinteren Aufnahme 85 unter dem Führungselement 80 entlanggeführt, sodass die Hauptfeder 40 zwischen den beiden Fersenkomponenten 51, 52 gelagert und zwischen dem Halter 30 und dem Führungselementes 80 eingeklemmt ist.

In der dargestellten, unbelasteten Situation des Prothesenfußeinsatzes 10 ist das Spannelement 90 leicht vorgespannt, sodass die beiden Fersenkomponenten 51, 52 klemmend zwischen dem Halter 30, der Hauptfeder 40 und dem Führungselement 80 gehalten sind. Bei einem Fersenstoß wird zumindest eine der Fersenkomponenten 51, 52 komprimiert und der Gurt 90 entspannt sich. Um zu verhindern, dass der Gurt 90 sich von dem Halter 30 und/oder dem Führungselement 80 löst, kann der Gurt 90 dort fixiert sein. Für eine bessere Führung und als Schutz gegen äußeren Einflüssen sind in dem Halter 30 und der hinteren Aufnahme 85 Ausnehmungen oder Nuten eingearbeitet, in denen der Gurt 90 geführt ist.

In der Explosionsdarstellung der Figur 13 sind die einzelnen Komponenten des Prothesenfußeinsatzes 10 dargestellt. Das Befestigungselement 20 ist als einschraubbarer Pyramidenadapter ausgebildet, der in ein Gewinde innerhalb des Halters 30 einschraubbar ist. In Gehrichtung hinter dem Befestigungselement 20 ist in dem Halter 30 die nutenförmige Ausnehmung 39 eingearbeitet, in der der Gurt 90 geführt ist. Um die Gurtspannung zu verändern, ist in dem Halter 30 ein Exzenter 300 gelagert. Der Exzenter 300 ist von der Rückseite des Halters 30 zugänglich und ermöglicht durch eine Verdrehung ausgehend von einer Ausgangsstellung eine Veränderung der Gurtspannung.

Die Kappe 45 ist als Rahmen aufgebaut, der medial und lateral sowie an der Rückseite proximal und distal von einer Auflage vorsteht, um eine Führung für die Fersenkomponente 51, 52 bereitzustellen. Innerhalb des Rahmens können als Auflage eine Platte, ein nach innen vorstehender Rahmen, mehrere Zierelemente oder ein Schlitz ausgebildet sein. Somit ist es möglich, dass die Kappe 45 auf der Hauptfeder 40 aufgelegt, die Hauptfeder 40 auf die Kappe 45 aufgelegt oder das hintere Ende der Hauptfeder 40 in den Schlitz der Kappe 45 eingeführt werden kann. Eine Befestigung der Kappe 45 an dem hinteren Ende der Hauptfeder 40 kann kraftschlüssig, formschlüssig und/oder stoffschlüssig erfolgen.

An dem vorderen Ende des Trägers 30 ist die Bohrung für die Aufnahme des Achsbolzens 112 zu erkennen, der in Muffen 114 innerhalb von Bohrungen in dem Träger 600 gelagert ist. Der Träger 600 ist brückenartig aufgebaut und weist zwei Auflagebereiche auf, sodass ein Kontakt zwischen dem Träger 600 und der Hauptfeder 40 über die beiden Zwischenstücke 71, 72 beabstandet zueinander erfolgt. Die Krafteinleitung in die Hauptfeder 40 bei einer Druckbelastung von oben erfolgt somit in Längserstreckung der Hauptfeder 40 beabstandeten zueinander, sodass der Halter 30 über den Träger 600 und die Zwischenstücke 71, 72 über eine 2-Punkt-Lagerung auf der Hauptfeder 40 abgestützt wird.

Die zu den Zwischenstücken 71, 72 in Längserstreckung beabstandeten Lagerpunkte der Hauptfeder 40 an ihren vorderen Ende und dem hinteren Ende auf dem Führungselement 80 und der distalen Fersenkomponente 52 bilden somit insgesamt eine 4-Punkt-Lagerung der Hauptfeder 40 aus.

Die beiden Fersenkomponente 51, 52 sind bevorzugt als Schaumelemente oder Schaumblöcke ausgebildet und können unterschiedliche Elastizitäten und Dämpfungseigenschaften aufweisen. Die Fersenkomponenten 51, 52 können auswechselbar, insbesondere zerstörungsfrei auswechselbar innerhalb des Prothesenfußeinsatzes 10 gelagert sein. Nach Entfernen des Spannelementes 90 kann beispielsweise der Halter 30 um das Gelenk 110 nach oben geklappt und die proximale Fersenkomponente 51 entnommen werden. Korrespondierend geschieht dies mit der distalen Fersenkomponente 52.

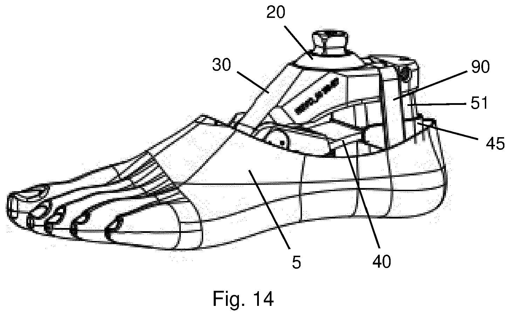

In der Figur 14 ist der zusammengebaute Prothesenfußeinsatz 10 gemäß Figur 12 mit einer Fußkosmetik 5 versehen, die der Form eines natürlichen Fußes nachgebildet ist. Die Fußkosmetik 5 dient insbesondere zum Schutz der mechanischen Komponenten des Prothesenfußeinsatzes 10 vor äußeren Einflüssen, zum Schutz eines Schuhes, in dem der Prothesenfußeinsatz 10 getragen wird sowie der Umgebung. Damit keine Schäden oder Verletzungen durch scharfkantige oder harte Komponenten des Prothesenfußeinsatzes 10 erfolgen, ist die Prothesenkosmetik vorteilhafterweise einem weichen, nachgiebigem Material, beispielsweise einem Polyurethan, einem Polyethylen oder einem Silikonwerkstoff oder einer Kombination mehrerer Werkstoffe ausgebildet.

## Patentansprüche

1. Prothesenfußeinsatz (10) mit einer proximalen Befestigungseinrichtung (20) zum Festlegen des Prothesenfußeinsatzes (10) an einer Proximalkomponente (2), einem distal zu der Befestigungseinrichtung (20) angeordneten und mit der Befestigungseinrichtung (20) gekoppelten Halter (30), einem elastischen Fersenelement (50), das an dem Halter (30) angeordnet ist und einer Hauptfeder (40), die sich in einen Vorderfußbereich erstreckt und mit dem Halter (30) gekoppelt ist, wobei die Hauptfeder (40) an dem Fersenelement (50) zwischen einer proximalen Fersenkomponente (51) und einer distalen Fersenkomponente (52) gelagert ist, **dadurch gekennzeichnet, dass** das Fersenelement (50) über zumindest ein Spannelement (90) mit dem Halter (30) gekoppelt ist, das das Fersenelement (50) in einer unbelasteten Ausgangsstellung in einer komprimierten Stellung hält.

2. Prothesenfußeinsatz nach Anspruch 1, **dadurch gekennzeichnet, dass** die distale Fersenkomponente (52) starr ausgebildet und die proximale Fersenkomponente (51) an dem Halter (30) und der Hauptfeder (40) festgelegt ist.

3. Prothesenfußeinsatz nach Anspruch 1, **dadurch gekennzeichnet, dass** die Hauptfeder (40) nicht Zugkräfte übertragend mit der proximalen Fersenkomponente (51) gekoppelt ist.

4. Prothesenfußeinsatz nach einem der voranstehenden Ansprüche, **dadurch gekennzeichnet, dass** die distale Fersenkomponente (52) härter als die proximale Fersenkomponente (51) ist.

5. Prothesenfußeinsatz nach einem der voranstehenden Ansprüche, **dadurch gekennzeichnet, dass** die Hauptfeder (40) zwischen der proximalen und der distalen Fersenkomponente (51, 52) eingebettet ist.

6. Prothesenfußeinsatz nach einem der voranstehenden Ansprüche, **dadurch gekennzeichnet, dass** die Hauptfeder (40) als Blattfeder ausgebildet ist.

7. Prothesenfußeinsatz nach einem der voranstehenden Ansprüche, **dadurch gekennzeichnet, dass** der Halter (30) einen in Richtung auf den Vorderfußbereich über das Befestigungselement (20) hervorstehenden Vorsprung (34) aufweist, der sich auf der Hauptfeder (40) abstützt.

8. Prothesenfußeinsatz nach Anspruch 7, **dadurch gekennzeichnet, dass** sich der Halter (30) an zwei in Längserstreckung der Hauptfeder (40) zueinander beabstandeten Bereichen (71, 72) auf der Hauptfeder (40) abstützt.

9. Prothesenfußeinsatz nach einem der Ansprüche 7 oder 8, **dadurch gekennzeichnet, dass** der Halter (30) sich über eine Zwischenfeder (60) oder Zwischenplatte auf der Hauptfeder (40) abstützt.

10. Prothesenfußeinsatz nach einem der voranstehenden Ansprüche, **dadurch gekennzeichnet, dass** an dem Fersenelement (50) ein Führungselement (80) befestigt ist, das an der Hauptfeder (40) gelagert ist.

11. Prothesenfußeinsatz nach Anspruch 10, **dadurch gekennzeichnet, dass** das Führungselement (80) an dem frontalen Ende des Hauptfeder (40) befestigt ist.

12. Prothesenfußeinsatz nach Anspruch 10 oder 11, **dadurch gekennzeichnet, dass** das Führungselement (80) eine Verlagerung des Fersenelementes (50) in medial-lateral-Richtung reduziert oder sperrt und eine Kompression oder Expansion des Fersenelementes (50) zulässt.

13. Prothesenfußeinsatz nach einem der voranstehenden Ansprüche, **dadurch gekennzeichnet, dass** das Spannelement (90) distal der distalen Fersenkomponente (52) geführt ist.

14. Prothesenfußeinsatz nach einem der voranstehenden Ansprüche, **dadurch gekennzeichnet, dass** das Spannelement (90) bei einer Vorderfußbelastung die distale Fersenkomponente (52) gegen die Hauptfeder (40) spannt.

15. Prothesenfußeinsatz nach einem der voranstehenden Ansprüche, **dadurch gekennzeichnet, dass** an der distalen Fersenkomponente (52) oder an dem Führungselement (80) ein Sohlenelement (100) angeordnet ist.

16. Prothesenfußeinsatz nach einem der voranstehenden Ansprüche, **dadurch gekennzeichnet, dass** der Halter (30) über ein Gelenk (110) mit der Hauptfeder (40), Zwischenfeder (60) oder Zwischenplatte gekoppelt ist.

17. Prothesenfußeinsatz nach Anspruch 16, **dadurch gekennzeichnet, dass** das Gelenk (110) im Mittelfußbereich angeordnet ist.

18. Prothesenfußeinsatz nach einem der voranstehenden Ansprüche, **dadurch gekennzeichnet, dass** an dem frontalen Ende der Hauptfeder (40) ein Zehenelement (120) befestigt ist.

19. Prothesenfußeinsatz nach einem der voranstehenden Ansprüche, **dadurch gekennzeichnet, dass** die Fersenkomponenten (51, 52) aus einem Schaumwerkstoff, Hohlkörper, Elastomerelement, Carbonelement, Elastomerelement mit Kavität als Pumpkammer und/oder Schraubenfederelementen bestehen.

20. Prothesenfußeinsatz nach einem der voranstehenden Ansprüche, **dadurch gekennzeichnet, dass** die Federn (40, 60, 120) als gerade Blattfedern ausgebildet sind.

21. Prothesenfußeinsatz nach einem der voranstehenden Ansprüche, **dadurch gekennzeichnet, dass** zwischen dem Halter (30) und der Hauptfeder (40) ein Dämpfer (200) oder Aktuator angeordnet ist.

22. Prothesenfußeinsatz nach Anspruch 21, **dadurch gekennzeichnet, dass** der Dämpfer (200) sperrbar ist.

23. Prothesenfußeinsatz nach Anspruch 21 oder 22, **dadurch gekennzeichnet, dass** der Dämpfer (200) ein Folgeventil beinhaltet.

24. Prothesenfußeinsatz nach einem der voranstehenden Ansprüche, **dadurch gekennzeichnet, dass** der Halter (30) verstellbar ausgebildet ist, um einen proximal-distal-Abstand der Befestigungseinrichtung (20) zu der Hauptfeder (40) einzustellen.

25. Prothesenfußeinsatz nach einem der voranstehenden Ansprüche, **dadurch gekennzeichnet, dass** die Befestigungseinrichtung (20) verschieblich, gelenkig oder drehbar an dem Halter (30) gelagert ist.

## Claims

1. A prosthetic foot insert (10) with a proximal fastening device (20) for securing the prosthetic foot insert (10) to a proximal component (2), a holder (30) arranged distally with respect to the fastening device (20) and coupled to the fastening device (20), an elastic heel element (50) which is arranged on the holder (30), and a main spring (40) which extends into a forefoot region and is coupled to the holder (30), **characterized in that** the main spring (40) is mounted on the heel element (50) between a proximal heel component (51) and a distal heel component (52), **characterized in that** the heel element (50) is coupled to the holder (30) via at least one tensioning element (90) that holds the heel element (50) in a compressed position.

2. The prosthetic foot insert as claimed in claim 1, **characterized in that** the distal heel component (52) is rigid, and the proximal heel component (51) is secured to the holder (30) and the main spring (40).

3. The prosthetic foot insert as claimed in claim 1, **characterized in that** the main spring (40) is coupled to the proximal heel component (51) in a manner not transmitting tensile forces.

4. The prosthetic foot insert as claimed in one of the preceding claims, **characterized in that** the distal heel component (52) is harder than the proximal heel component (51).

5. The prosthetic foot insert as claimed in one of the preceding claims, **characterized in that** the main spring (40) is embedded between the proximal and the distal heel component (51, 52).

6. The prosthetic foot insert as claimed in one of the preceding claims, **characterized in that** the main spring (40) is designed as a leaf spring.

7. The prosthetic foot insert as claimed in one of the preceding claims, **characterized in that** the holder (30) has a projection (34) which protrudes in the direction of the forefoot region past the fastening element (20) and which is supported on the main spring (40).

8. The prosthetic foot insert as claimed in claim 7, **characterized in that** the holder (30) is supported on the main spring (40) at two regions (71, 72) spaced apart from each other in the longitudinal extent of the main spring (40).

9. The prosthetic foot insert as claimed in either of claims 7 and 8, **characterized in that** the holder (30) is supported on the main spring (40) via an intermediate spring (60) or intermediate plate.

10. The prosthetic foot insert as claimed in one of the preceding claims,
**characterized in that** a guide element (80) is fastened to the heel element (50) and is mounted on the main spring (40).

11. The prosthetic foot insert as claimed in claim 10, **characterized in that** the guide element (80) is fastened to the front end of the main spring (40).

12. The prosthetic foot insert as claimed in claim 10 or 11, **characterized in that** the guide element (80) reduces or blocks a displacement of the heel element (50) in the medial-lateral direction and permits a compression or expansion of the heel element (50).

13. The prosthetic foot insert as claimed in one of the preceding claims,
**characterized in that** the tensioning element (90) is guided distally with respect to the distal heel component (52).

14. The prosthetic foot insert as claimed in one of the preceding claims, **characterized in that** the tensioning element (90) tensions the distal heel component (52) against the main spring (40) when the forefoot is loaded.

15. The prosthetic foot insert as claimed in one of the preceding claims, **characterized in that** a sole element (100) is arranged on the distal heel component (52) or on the guide element (80).

16. The prosthetic foot insert as claimed in one of the preceding claims,
**characterized in that** the holder (30) is coupled to the main spring (40), intermediate spring (60) or intermediate plate via a joint (110).

17. The prosthetic foot insert as claimed in claim 16, **characterized in that** the joint (110) is arranged in the midfoot region.

18. The prosthetic foot insert as claimed in one of the preceding claims,
**characterized in that** a toe element (120) is fastened to the front end of the main spring (40).

19. The prosthetic foot insert as claimed in one of the preceding claims,
**characterized in that** the heel components (51, 52) are made from a foam material, hollow body, elastomer element, carbon element, elastomer element with cavity as pump chamber and/or helical spring elements.

20. The prosthetic foot insert as claimed in one of the preceding claims,
**characterized in that** the springs (40, 60, 120) are designed as straight leaf springs.

21. The prosthetic foot insert as claimed in one of the preceding claims, **characterized in that** a damper (200) or actuator is arranged between the holder (30) and the main spring (40).

22. The prosthetic foot insert as claimed in claim 21, **characterized in that** the damper (200) is lockable.

23. The prosthetic foot insert as claimed in claim 21 or 22, **characterized in that** the damper (200) contains a sequence valve.

24. The prosthetic foot insert as claimed in one of the preceding claims,
**characterized in that** the holder (30) is designed to be adjustable in order to set a proximal-distal distance of the fastening device (20) from the main spring (40).

25. The prosthetic foot insert as claimed in one of the preceding claims,
**characterized in that** the fastening device (20) is mounted in a displaceable, articulated or rotatable manner on the holder (30).

## Revendications

1. Insert de pied prothétique (10) comprenant un dispositif de fixation proximal (20) destiné à fixer l'insert de pied prothétique (10) à un composant proximal (2), un support (30) disposé distalement par rapport au dispositif de fixation (20) et couplé au dispositif de fixation (20), un élément de talon (50) élastique disposé sur le support (30), et un ressort principal (40) qui s'étend dans une zone d'avant-pied et qui est couplé au support (30), le ressort principal (40) étant supporté sur l'élément de talon (50) entre un composant de talon proximal (51) et un composant de talon distal (52),
**caractérisé en ce que** l'élément de talon (50) est couplé au support (30) par l'intermédiaire d'au moins un élément de serrage (90) qui, dans une position initiale non chargée, maintient l'élément de talon (50) dans une position comprimée.

2. Insert de pied prothétique selon la revendication 1,
**caractérisé en ce que** le composant de talon distal (52) est réalisé rigide, et le composant de talon proximal (51) est fixé au support (30) et au ressort principal (40).

3. Insert de pied prothétique selon la revendication 1,
**caractérisé en ce que** le ressort principal (40) est couplé au composant de talon proximal (51) de manière à ne pas transmettre de forces de traction.

4. Insert de pied prothétique selon l'une des revendications précédentes,
**caractérisé en ce que** le composant de talon distal (52) est plus dur que le composant de talon proximal (51).

5. Insert de pied prothétique selon l'une des revendications précédentes,
**caractérisé en ce que** le ressort principal (40) est intégré entre les composants de talon proximal et distal (51, 52).

6. Insert de pied prothétique selon l'une des revendications précédentes,
**caractérisé en ce que** le ressort principal (40) est conçu comme un ressort à lames.

7. Insert de pied prothétique selon l'une des revendications précédentes,
**caractérisé en ce que** le support (30) présente une saillie (34) qui dépasse de l'élément de fixation (20) en direction de la zone d'avant-pied et qui s'appuie sur le ressort principal (40).

8. Insert de pied prothétique selon la revendication 7,
**caractérisé en ce que** le support (30) s'appuie sur le ressort principal (40) au niveau de deux zones (71, 72) espacées l'une de l'autre dans l'extension longitudinale du ressort principal (40).

9. Insert de pied prothétique selon l'une des revendications 7 ou 8,
**caractérisé en ce que** le support (30) s'appuie sur le ressort principal (40) par l'intermédiaire d'un ressort intermédiaire (60) ou d'une plaque intermédiaire.

10. Insert de pied prothétique selon l'une des revendications précédentes, **caractérisé en ce qu'**un élément de guidage (80) est fixé à l'élément de talon (50) et est supporté sur le ressort principal (40).

11. Insert de pied prothétique selon la revendication 10,
**caractérisé en ce que** l'élément de guidage (80) est fixé à l'extrémité frontale du ressort principal (40).

12. Insert de pied prothétique selon la revendication 10 ou 11,
**caractérisé en ce que** l'élément de guidage (80) réduit ou bloque un déplacement de l'élément de talon (50) dans la direction médio-latérale et autorise une compression ou une expansion de l'élément de talon (50).

13. Insert de pied prothétique selon l'une des revendications précédentes, **caractérisé en ce que** l'élément de serrage (90) est guidé distalement par rapport au composant de talon distal (52).

14. Insert de pied prothétique selon l'une des revendications précédentes, **caractérisé en ce que** l'élément de serrage (90) serre le composant de talon distal (52) contre le ressort principal (40) lors d'une sollicitation de l'avant-pied.

15. Insert de pied prothétique selon l'une des revendications précédentes, **caractérisé en ce qu'**un élément de semelle (100) est disposé sur le composant de talon distal (52) ou sur l'élément de guidage (80).

16. Insert de pied prothétique selon l'une des revendications précédentes, **caractérisé en ce que** le support (30) est couplé au ressort principal (40), au ressort intermédiaire (60) ou à la plaque intermédiaire par une articulation (110).

17. Insert de pied prothétique selon la revendication 16,
**caractérisé en ce que** l'articulation (110) est disposée dans la zone métatarsienne.

18. Insert de pied prothétique selon l'une des revendications précédentes, **caractérisé en ce qu'**un élément d'orteils (120) est fixé à l'extrémité frontale du ressort principal (40).

19. Insert de pied prothétique selon l'une des revendications précédentes, **caractérisé en ce que** les composants de talon (51, 52) sont constitués d'un matériau en mousse, d'un corps creux, d'un élément en élastomère, d'un élément en carbone, d'un élément en élastomère avec une cavité servant de chambre de pompage et/ou d'éléments de ressort hélicoïdal.

20. Insert de pied prothétique selon l'une des revendications précédentes, **caractérisé en ce que** les ressorts (40, 60, 120) sont formés comme des ressorts à lames droits.

21. Insert de pied prothétique selon l'une des revendications précédentes, **caractérisé en ce qu'**un amortisseur (200) ou un actionneur est disposé entre le support (30) et le ressort principal (40).

22. Insert de pied prothétique selon la revendication 21,
**caractérisé en ce que** l'amortisseur (200) peut être bloqué.

23. Insert de pied prothétique selon la revendication 21 ou 22,
**caractérisé en ce que** l'amortisseur (200) contient une soupape de séquence.

24. Insert de pied prothétique selon l'une des revendications précédentes, **caractérisé en ce que** le support (30) est conçu de manière réglable pour ajuster une distance proximale-distale du dispositif de fixation (20) par rapport au ressort principal (40).

25. Insert de pied prothétique selon l'une des revendications précédentes, **caractérisé en ce que** le dispositif de fixation (20) est supporté de manière coulissante, articulée ou rotative sur le support (30).
